# EUROPEAN PATENT APPLICATION

(11) **EP 2 218 717 A1**
(43) Date of publication of application: **18.08.2010**
(21) Application number: 09356010.0
(22) Date of filing: 17.02.2009
(51) Int. Cl.: C07D 401/12, C07D 417/12, A01N 43/40, A01N 43/56, A01N 43/78

(54) **Fungicidal N-((HET)Arylethyl)thiocarboxamide derivatives**

(71) Applicant: Bayer CropScience AG, 40789 Monheim (DE)
(72) Inventor: Desbordes, Philippe, 69006 Lyon (FR); Gary, Stéphanie, 69410 Champagne au Mont d'Or (FR); Grosjean-Cournoyer, Marie-Claire, 69250 Curis au Mont d'Or (FR); Rinolfi, Philippe, 69380 Chatillon d'Azergues (FR); Vors, Jean-Pierre, 69110 Sainte-Foy-Les-Lyon (FR)
(74) Representative: Guitton, Carole

(57) **Abstract**

The present invention relates to a N-((het)arylethyl)thiocarboxamide derivative of formula (I) wherein A represents a carbo-linked, unsaturated or partially saturated, substituted or non-substituted 5-membered heterocyclyl group; W represents a nitrogen atom or a substituted carbon atom; X, Z¹, Z² Z³, Z⁴, and Z⁵ represent various substituents, their process of preparation,the preparation of intermediate compounds, their use as fungicide active agents, particularly in the form of fungicide compositions and methods for the control of phytopathogenic fungi, notably of plants, using these compounds or compositions.

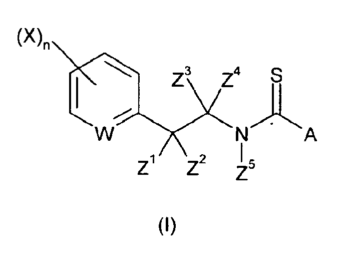

## Description

The present invention relates to fungicidal N-((het)arylethyl)thiocarboxamide derivatives, their process of preparation, their use as fungicides, particularly in the form of fungicidal compositions and methods for the control of phytopathogenic fungi of plants using these compounds or their compositions.

In international patent application WO-2005/085238 certain fungicidal N-(2-Pyridylethyl) carboxamide derivatives are generically embraced in a broad disclosure of numerous compounds of the following formula: 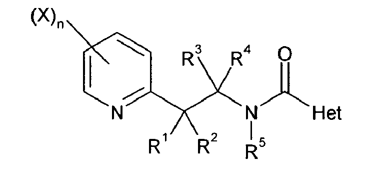 wherein Het can represent a 5-membered heterocyclic ring, R¹, R², R³ and R⁴ can represent various substituents among which a hydrogen atom, a halogen atom or a C₁-C₆-alkyl group, R⁵ can represent various substituents among which a C₃-C₇-cycloalkyl group, and X can represent various substituents among which a hydrogen atom, a halogen atom or a C₁-C₈-alkyl group and the like. However, this document does not disclose compounds wherein the carboxamide residue can be replace by a thiocarboxamide residue.

In international patent application WO-2005/058833 certain fungicidal N-(2-Pyridylethyl) carboxamide derivatives are generically embraced in a broad disclosure of numerous compounds of the following formula: 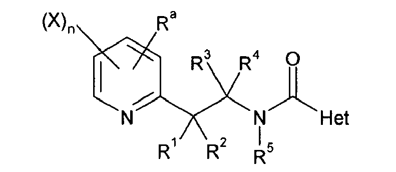 wherein Het can represent a 5-membered heterocyclic ring, R¹, R², R³ and R⁴ can represent various substituents among which a hydrogen atom, a halogen atom or a C₁-C₆-alkyl group, R⁵ can represent various substituents among which a C₃-C₇-cycloalkyl group, R^{a} can represent a C₁-C₆-halogenoalkyl group, and X can represent a hydrogen atom, a halogen atom or a C₁-C₆-alkyl group and a C₁-C₆-halogenoalkyl group. However, this document does not disclose compounds wherein the carboxamide residue can be replace by a thiocarboxamide residue.

In international patent application WO-2007/060164 certain fungicidal N-(2-phenylethyl) carboxamide derivatives are generically embraced in a broad disclosure of numerous compounds of the following formula: 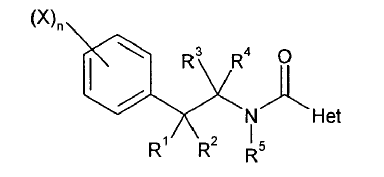 wherein Het can represent a 5-membered heterocyclic ring, R¹, R², R³ and R⁴ can represent a hydrogen atom or a C₁-C₆-alkyl group, R⁵ can represent various substituents among which a C₃-C₇-cycloalkyl group, and X can represent various substituents among which a hydrogen atom, a halogen atom or a C₁-C₈-alkyl group and the like. However, this document does not disclose compounds wherein the carboxamide residue can be replace by a thiocarboxamide residue.
In international patent application WO-2007/060166 certain fungicidal N-(2-phenylethyl) carboxamide derivatives are generically embraced in a broad disclosure of numerous compounds of the following formula: 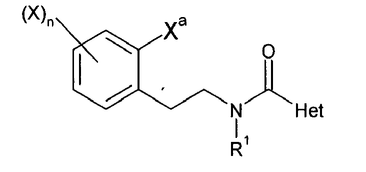 wherein Het can represent a 5-membered heterocyclic ring, R¹ can represent various substituents among which a C₃-C₇-cycloalkyl group, X can represent various substituents among which a hydrogen atom, a halogen atom or a C₁-C₈-alkyl group and the like and X^{a} can represent various substituents among which a halogen atom or a C₁-C₈-alkyl group and the like. However, this document does not disclose compounds wherein the carboxamide residue can be replace by a thiocarboxamide residue.

It is always of high-interest in the field of agrochemicals to use pesticidal compounds more active than the compounds already known by the man ordinary skilled in the art whereby reduced amounts of compound can be used whilst retaining equivalent efficacy.
Furthermore, the provision of new pesticidal compounds with a higher efficacy strongly reduces the risk of appearance of resistant strains in the fungi to be treated.
We have now found a new family of compounds which show enhanced fungicidal activity over the general known family of such compounds.

Accordingly, the present invention provides a N-((het)arylethyl)thiocarboxamide derivative of formula (I) 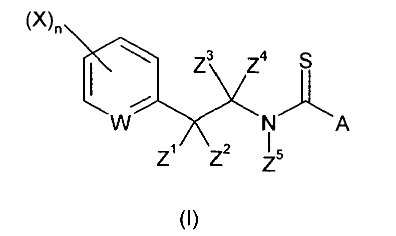 wherein
- A represents a carbo-linked, unsaturated or partially saturated, 5-membered heterocyclyl group that can be substituted by up to four groups R ;
- W represents a nitrogen atom or a carbon atom substituted by a hydrogen atom or by the group X ;
- n represents 0, 1, 2, 3 or 4 ;
- X represents a halogen atom, a nitro group, a cyano group, a hydroxy group, an amino group, a sulfanyl group, a pentafluoro-λ⁶-sulfanyl group, a formyl group, a formyloxy group, a formylamino group, a carboxy group, a carbamoyl group, a N-hydroxycarbamoyl group, a carbamate group, a (hydroxyimino)-C₁-C₆-alkyl group, a C₁-C₈-halogenoalkyl having 1 to 5 halogen atoms, a C₁-C₈-alkyl, a C₁-C₈-arylalkyl, a (C₃-C₇-cycloalkyl)-C₁-C₈-alkyl, a C₂-C₈-alkenyl, a C₂-C₈-arylalkenyl, a (C₃-C₇-cycloalkyl)-C₂-C₈-alkenyl, a C₂-C₈-alkynyl, a C₂-C₈-arylalkynyl, a (C₃-C₇-cycloalkyl)-C₂-C₈-alkynyl, a C₁-C₈-alkylamino, a di-C₁-C₈-alkylamino, a C₁-C₈-alkoxy, a C₁-C₈-halogenoalkoxy having 1 to 5 halogen atoms, a C₁-C₈-alkylsulfanyl, a C₁-C₈-halogenoalkylsulfanyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₃-C₈-cycloalkyl, a C₃-C₈-halogenocycloalkyl having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a N-C₁-C₈-alkyloxycarbamoyl, a C₁-C₈-alkoxycarbamoyl, a N-C₁-C₈-alkyl-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl, a C₁-C₈-halogenoalkylsulphonyl having 1 to 5 halogen atoms, a C₁-C₆-alkoxyimino, a (C₁-C₆-alkoxyimino)-C₁-C₆-alkyl, a (C₁-C₆-alkenyloxyimino)-C₁-C₆-alkyl, a (C₁-C₆-alkynyloxyimino)-C₁-C₆-alkyl, a (benzyloxyimino)-C₁-C₆-alkyl, a tri(C₁-C₈)alkylsilyl, a tri(C₁-C₈)alkylsilyl-C₁-C₈-alkyl, a benzyloxy that can be substituted by up to 5 groups Q ; a benzylsulfanyl that can be substituted by up to 5 groups Q ; a benzylamino that can be substituted by up to 5 groups Q ; a phenyl that can be substituted by up to 5 groups Q ; a naphtyl that can be substituted by up to 6 groups Q ; a phenoxy that can be substituted by up to 5 groups Q ; a phenylamino that can be substituted by up to 5 groups Q ; a phenylsulfanyl that can be substituted by up to 5 groups Q ; a phenylmethylene that can be substituted by up to 7 groups Q ; a pyridinyl that can be substituted by up to four groups Q or a pyridinyloxy that can be substituted by up to four groups Q ;
- Z¹ and Z² independently represent a hydrogen atom, a halogen atom, a cyano group, a hydroxy group, an amino group, a sulfanyl group, a formyl group, a formyloxy group, a formylamino group, a carboxy group, a carbamoyl group, a N-hydroxycarbamoyl group, a carbamate group, a (hydroxyimino)-C₁-C₈-alkyl group, a C₁-C₈-alkyl, a C₂-C₈-alkenyl, a C₂-C₈-alkynyl, a C₁-C₈-alkylamino, a di-C₁-C₈-alkylamino, a C₁-C₈-alkoxy, a C₁-C₈-halogenoalkyl having 1 to 5 halogen atoms, a C₁-C₈-halogenoalkoxy having 1 to 5 halogen atoms, a C₁-C₈-alkylsulfanyl, a C₁-C₈-halogenoalkylsulfanyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₃-C₇-cycloalkyl, a C₃-C₇-halogenocycloalkyl having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a (N-C₁-C₈-alkyl)oxycarbamoyl, a C₁-C₈-alkoxycarbamoyl, a (N-C₁-C₈-alkyl)-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl or a C₁-C₈-halogenoalkylsulphonyl having 1 to 5 halogen atoms,
- Z³ and Z⁴ independently represent a hydrogen atom, a halogen atom, a cyano group, a hydroxy group, an amino group, a sulfanyl group, a formyl group, a formyloxy group, a formylamino group, a carboxy group, a carbamoyl group, a N-hydroxycarbamoyl group, a carbamate group, a (hydroxyimino)-C₁-C₈-alkyl group, a C₁-C₈-alkyl, a C₂-C₈-alkenyl, a C₂-C₈-alkynyl, a C₁-C₈-alkylamino, a di-C₁-C₈-alkylamino, a C₁-C₈-alkoxy, a C₁-C₈-halogenoalkyl having 1 to 5 halogen atoms, a C₁-C₈-halogenoalkoxy having 1 to 5 halogen atoms, a C₁-C₈-alkylsulfanyl, a C₁-C₈-halogenoalkylsulfanyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₃-C₇-cycloalkyl, a C₃-C₇-halogenocycloalkyl having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a (N-C₁-C₈-alkyl)oxycarbamoyl, a C₁-C₈-alkoxycarbamoyl, a (N-C₁-C₈-alkyl)-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl or a C₁-C₈-halogenoalkylsulphonyl having 1 to 5 halogen atoms, With the provisio that when W is a nitrogen atom, then a least one of the four substituents Z₁, Z₂, Z₃ or Z₄ is not a hydrogen atom;
- Z⁵ represents a non-substituted C₃-C₇-cycloalkyl or a C₃-C₇-cycloalkyl substituted by up to 10 atoms or groups that can be the same or different and that can be selected in the list consisting of halogen atoms ; cyano ; C₁-C₈-alkyl ; C₁-C₈-halogenoalkyl comprising up to 9 halogen atoms that can be the same or different ; C₁-C₈-alkoxy ; C₁-C₈-halogenoalkoxy comprising up to 9 halogen atoms that can be the same or different ; C₁-C₈-alkoxycarbonyl ; C₁-C₈-halogenoalkoxycarbonyl comprising up to 9 halogen atoms that can be the same or different ; C₁-C₈-alkylaminocarbonyl ; or di-C₁-C₈-alkylaminocarbonyl ;
- Q independently represents a halogen atom ; cyano ; nitro ; C₁-C₈-alkyl ; C₁-C₈-alkoxy ; C₁-C₈-alkylsulfanyl ; C₁-C₈-halogenoalkyl comprising up to 9 halogen atoms that can be the same or different ; C₁-C₈-halogenoalkoxy comprising up to 9 halogen atoms that can be the same or different ; tri(C₁-C₈)alkylsilyl and tri(C₁-C₈)alkylsilyl-C₁-C₈-alkyl ; C₁-C₈-alkoxyimino ; or (C₁-C₈-alkoxyimino)- C₁-C₈-alkyl ;
- R independently represents a hydrogen atom ; halogen atom ; cyano ; nitro ; amino ; sulfanyl ; pentafluoro-λ-6-sulfanyl ; C₁-C₈-alkylamino ; di-C₁-C₈-alkylamino ; tri(C₁-C₈-alkyl)silyl ; C₁-C₈-alkylsulfanyl ; C₁-C₈-halogenoalkylsulfanyl comprising up to 9 halogen atoms that can be the same or different ; C₁-C₈-alkyl ; C₁-C₈-halogenoalkyl comprising up to 9 halogen atoms that can be the same or different ; C₂-C₈-alkenyl ; C₂-C₈-halogenoalkenyl comprising up to 9 halogen atoms that can be the same or different ; C₂-C₈-alkynyl ; C₂-C₈-halogenoalkynyl comprising up to 9 halogen atoms that can be the same or different ; C₁-C₈-alkoxy ; C₁-C₈-halogenoalkoxy comprising up to 9 halogen atoms that can be the same or different ; C₂ C₈-alkenyloxy ; C₂-C₈-alkynyloxy ; C₃-C₇-cycloalkyl ; C₃-C₇-cycloalkyl- C₁-C₈-alkyl ; C₁-C₈-alkylsulphinyl ; C₁-C₈-alkylsulphonyl ; C₁-C₈alkoxyimino ; (C₁-C₈-alkoxyimino)-C₁-C₈-alkyl ; (benzyloxyimino)-C₁-C₈-alkyl ; phenoxy ; benzyloxy ; benzylsulfanyl ; benzylamino ; naphtyl ; halogenophenoxy comprising up to 9 halogen atoms that can be the same or different ; C₁-C₈-alkylcarbonyl ; C₁-C₈-halogenoalkylcarbonyl comprising up to 9 halogen atoms that can be the same or different ; C₁-C₈-alkoxycarbonyl ; C₁-C₈-halogenoalkoxycarbonyl comprising up to 9 halogen atoms that can be the same or different; C₁-C₈-alkylaminocarbonyl ; or di-C₁-C₈-alkylaminocarbonyl ;
as well as its salts, N-oxydes, metallic complexes, metalloidic complexes and optically active isomers.

For the compounds according to the invention, the following generic terms are generally used with the following meanings:
- halogen means fluorine, bromine, chlorine or iodine.
   carboxy means -C(=O)OH ;
   carbonyl means -C(=O)- ;
   carbamoyl means -C(=O)NH₂;
   N-hydroxycarbamoyl means -C(=O)NHOH ;
   SO represents a sulfoxyde group ;
   SO₂ represents a sulfone group ;
- an alkyl group, an alkenyl group and an alkynyl group as well as moieties containing these terms, can be linear or branched; and heteroatom means sulphur, nitrogen or oxygen.
- the aryl moeity contained in an arylalkyl group, an arylalkenyl group and an arylalkynyl group, can be a phenyl group that can be substituted by up to 5 groups Q ; a naphtyl group that can be substituted by up to 6 groups Q or a pyridyl group that can be substituted by up to 4 groups Q;
- in the case of an amino group or the amino moiety of any other amino-comprising group, substituted by two substituents that can be the same or different, the two substituents together with the nitrogen atom to which they are linked can form a heterocyclyl group, preferably a 5- to 7-membered heterocyclyl group, that can be substituted or that can include other hetero atoms, for example a morpholino group or piperidinyl group.

Any of the compounds of the present invention can exist in one or more optical or chiral isomer forms depending on the number of asymmetric centres in the compound. The invention thus relates equally to all the optical isomers and to their racemic or scalemic mixtures (the term "scalemic" denotes a mixture of enantiomers in different proportions) and to the mixtures of all the possible stereoisomers, in all proportions. The diastereoisomers and/or the optical isomers can be separated according to the methods which are known *per se* by the man ordinary skilled in the art.
Any of the compounds of the present invention can also exist in one or more geometric isomer forms depending on the number of double bonds in the compound. The invention thus relates equally to all geometric isomers and to all possible mixtures, in all proportions. The geometric isomers can be separated according to general methods, which are known *per se* by the man ordinary skilled in the art.
Any of the compounds of formula (I) wherein X represents a hydroxy, a sulfanyl group or an amino group may be found in its tautomeric form resulting from the shift of the proton of said hydroxy, sulfanyl or amino group. Such tautomeric forms of such compounds are also part of the present invention. More generally speaking, all tautomeric forms of compounds of formula (I) wherein X represents a hydroxy, a sulfanyl group or an amino group, as well as the tautomeric forms of the compounds which can optionally be used as intermediates in the preparation processes and which will be defined in the description of these processes, are also part of the present invention.

Preferred compounds according to the invention are compounds of formula (I) wherein A is selected in the list consisting of:
- a heterocycle of formula (A¹) 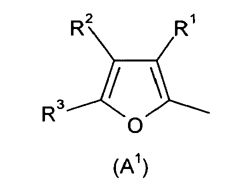
wherein :
R¹ to R³ that can be the same or different represent a hydrogen atom ; a halogen atom ; C₁-C₅-alkyl ; C₁-C₅-halogenoalkyl comprising up to 9 halogen atoms that can be the same or different ; C₁-C₅-alkoxy or C₁-C₅-halogenoalkoxy comprising up to 9 halogen atoms that can be the same or different;
   - a heterocycle of formula (A²) 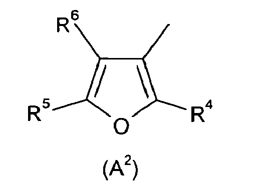
wherein :
R⁴ to R⁶ that can be the same or different represent a hydrogen atom ; a halogen atom ; C₁-C₅-alkyl ; C₁-C₅-halogenoalkyl comprising up to 9 halogen atoms that can be the same or different ; C₁-C₅-alkoxy or C₁-C₅-halogenoalkoxy comprising up to 9 halogen atoms that can be the same or different ;
   - a heterocycle of formula (A³) 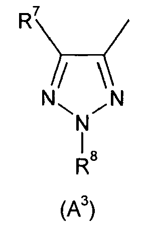
wherein :
R⁷ represents a hydrogen atom ; a halogen atom ; C₁-C₅-alkyl ; C₁-C₅-halogenoalkyl comprising up to 9 halogen atoms that can be the same or different ; C₁-C₅-alkoxy or C₁-C₅-halogenoalkoxy comprising up to 9 halogen atoms that can be the same or different;
R⁸ represents a hydrogen atom or a C₁-C₅-alkyl ;
   - a heterocycle of formula (A⁴) 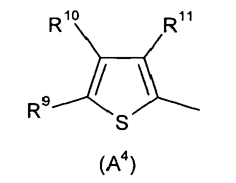
wherein :
R⁹ to R¹¹ that can be the same or different represent a hydrogen atom ; a halogen atom ; C₁-C₅-alkyl ; amino ; C₁-C₅-alkoxy ; C₁-C₅-alkylsulphanyl ; C₁-C₅-halogenoalkyl comprising up to 9 halogen atoms that can be the same or different or C₁-C₅-halogenoalkoxy comprising up to 9 halogen atoms that can be the same or different;
   - a heterocycle of formula (A⁵) 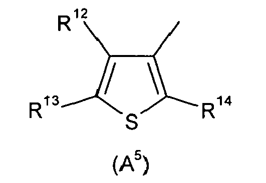
wherein :
R¹² and R¹³ that can be the same or different represent a hydrogen atom ; a halogen atom ; C₁-C₅-alkyl ; C₁-C₅-alkoxy ; amino ; C₁-C₅-halogenoalkyl comprising up to 9 halogen atoms that can be the same or different or C₁-C₅-halogenoalkoxy comprising up to 9 halogen atoms that can be the same or different;
R¹⁴ represents a hydrogen atom ; a halogen atom ; C₁-C₅-alkyl ; C₁-C₅-alkoxy ; amino ; C₁-C₅-halogenoalkyl comprising up to 9 halogen atoms that can be the same or different or C₁-C₅-halogenoalkoxy comprising up to 9 halogen atoms that can be the same or different;
   - a heterocycle of formula (A⁶) 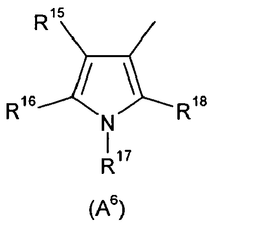
wherein :
R¹⁵ represents a hydrogen atom ; a halogen atom ; a cyano ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different;
R¹⁶ and R¹⁸ that can be the same or different represent a hydrogen atom ; a halogen atom ; C₁-C₅-alkoxycarbonyl ; C₁-C₅-alkyl ; C₁-C₅-halogenoalkoxy comprising up to 9 halogen atoms that can be the same or different or C₁-C₅-halogenoalkyl comprising up to 9 halogen atoms that can be the same or different;
R¹⁷ represent a hydrogen atom or C₁-C₅-alkyl ;
   - a heterocycle of formula (A⁷) 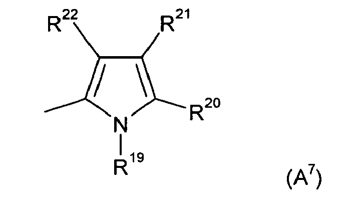
wherein :
R¹⁹ represents a hydrogen atom or a C₁-C₅-alkyl
R²⁰ to R²² that can be the same or different represent a hydrogen atom ; a halogen atom ; C₁-C₅-alkyl or C₁-C₅-halogenoalkyl comprising up to 9 halogen atoms that can be the same or different ;
   - a heterocycle of formula (A⁸) 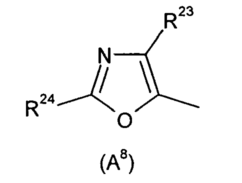
wherein :
R²³ represents a hydrogen atom ; a halogen atom ; C₁-C₅-alkyl or C₁-C₅-halogenoalkyl comprising up to 9 halogen atoms that can be the same or different;
R²⁴ represents a hydrogen atom or C₁-C₅-alkyl or C₁-C₅-halogenoalkyl comprising up to 9 halogen atoms that can be the same or different;
   - a heterocycle of formula (A⁹) 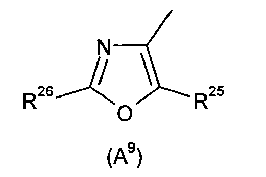
wherein :
R²⁵ represents a hydrogen atom ; a halogen atom ; C₁-C₅-alkyl or C₁-C₅-halogenoalkyl comprising up to 9 halogen atoms that can be the same or different;
R²⁶ represents a hydrogen atom ; C₁-C₅-alkyl or C₁-C₅-halogenoalkyl comprising up to 9 halogen atoms that can be the same or different;
   - a heterocycle of formula (A¹⁰) 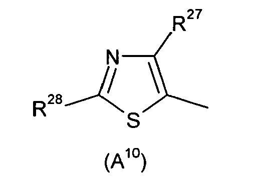
wherein :
R²⁷ represents a hydrogen atom ; a halogen atom ; C₁-C₅-alkyl or C₁-C₅-halogenoalkyl comprising up to 9 halogen atoms that can be the same or different;
R²⁸ represents a hydrogen atom ; a halogen atom ; C₁-C₅-alkyl ; C₁-C₅-halogenoalkyl comprising up to 9 halogen atoms that can be the same or different ; C₁-C₅-halogenoalkoxy comprising up to 9 halogen atoms that can be the same or different ; amino ; C₁-C₅-alkylamino or di(C₁-C₅-alkyl)amino ;
   - a heterocycle of formula (A¹¹) 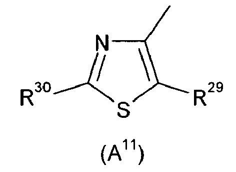
wherein :
R²⁹ represents a hydrogen atom ; a halogen atom ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different;
R³⁰ represents a hydrogen atom ; a halogen atom ; C₁-C₅-alkyl ; C₁-C₅-halogenoalkyl comprising up to 9 halogen atoms that can be the same or different ; C₁-C₅-halogenoalkoxy comprising up to 9 halogen atoms that can be the same or different ; amino ; C₁-C₅-alkylamino or di(C₁-C₅-alkyl)amino ;
   - a heterocycle of formula (A¹²) 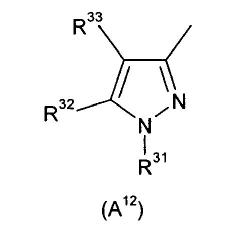
wherein :
R³¹ represents a hydrogen atom or a C₁-C₅-alkyl
R³² represents a hydrogen atom ; a halogen atom ; C₁-C₅-alkyl or C₁-C₅-halogenoalkyl comprising up to 9 halogen atoms that can be the same or different;
R³³ represents a hydrogen atom ; a halogen atom ; a nitro ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different;
   - a heterocycle of formula (A¹³) 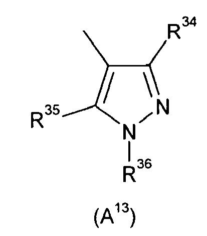
wherein :
R³⁴ represents a hydrogen atom ; a halogen atom ; C₁-C₅-alkyl ; C₃-C₅-cycloalkyl ; C₁-C₅-halogenoalkyl comprising up to 9 halogen atoms that can be the same or different ; C₁-C₅-alkoxy ; C₂-C₅-alkynyloxy or C₁-C₅-halogenoalkoxy comprising up to 9 halogen atoms that can be the same or different;
R³⁵ represents a hydrogen atom ; a halogen atom ; C₁-C₅-alkyl ; a cyano ; C₁-C₅-alkoxy ; C₁-C₅-alkylsulphanyl ; C₁-C₅-halogenoalkyl comprising up to 9 halogen atoms that can be the same or different ; C₁-C₅-halogenoalkoxy comprising up to 9 halogen atoms that can be the same or different; amino ; C₁-C₅-alkylamino or di(C₁-C₅-alkyl)amino ;
R³⁶ represents a hydrogen atom or C₁-C₅-alkyl ;
   - a heterocycle of formula (A¹⁴) 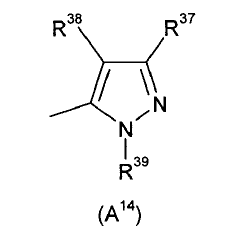
wherein :
R³⁷ and R³⁸ that can be the same or different represent a hydrogen atom ; a halogen atom ; C₁-C₅-alkyl ; C₁-C₅-halogenoalkyl comprising up to 9 halogen atoms that can be the same or different; C₁-C₅-alkoxy or a C₁-C₅- alkylsulphanyl ;
R³⁹ represents a hydrogen atom or C₁-C₅-alkyl ;
   - a heterocycle of formula (A¹⁵) 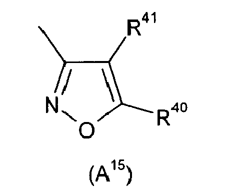
wherein :
R⁴⁰ and R⁴¹ that can be the same or different represent a hydrogen atom ; a halogen atom ; C₁-C₅-alkyl or C₁-C₅-halogenoalkyl comprising up to 9 halogen atoms that can be the same or different ;
   - a heterocycle of formula (A¹⁶) 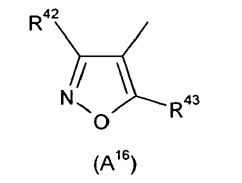
wherein :
R⁴² and R⁴³ that can be the same or different represent a hydrogen atom ; a halogen atom ; C₁-C₅-alkyl ; C₁-C₅-halogenoalkyl comprising up to 9 halogen atoms that can be the same or different or amino ;
   - a heterocycle of formula (A¹⁷) 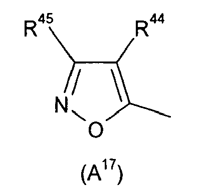
wherein :
R⁴⁴ and R⁴⁵ that can be the same or different represent a hydrogen atom ; a halogen atom ; C₁-C₅-alkyl or C₁-C₅-halogenoalkyl comprising up to 9 halogen atoms that can be the same or different ;
   - a heterocycle of formula (A¹⁸) 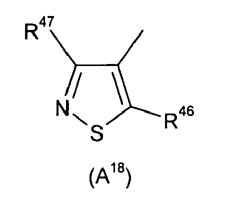
wherein :
R⁴⁷ represents a hydrogen atom ; a halogen atom ; C₁-C₅-alkyl or C₁-C₅-halogenoalkyl comprising up to 9 halogen atoms that can be the same or different;
R⁴⁶ represents a hydrogen atom ; a halogen atom ; C₁-C₅-alkyl ; C₁-C₅-halogenoalkyl comprising up to 9 halogen atoms that can be the same or different or C₁-C₅-alkylsulfanyl ;
   - a heterocycle of formula (A¹⁹) 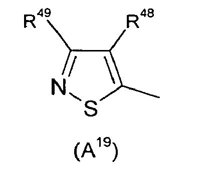
wherein :
R⁴⁹ and R⁴⁸ that can be the same or different represent a hydrogen atom ; a halogen atom ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different ;
   - a heterocycle of formula (A²⁰) 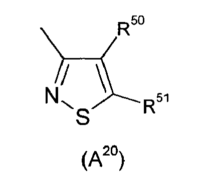
wherein :
R⁵⁰ and R⁵¹ that can be the same or different represent a hydrogen atom ; a halogen atom ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different ;
   - a heterocycle of formula (A²¹) 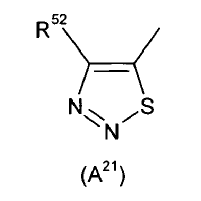
wherein :
R⁵² represents a hydrogen atom ; a halogen atom ; C₁-C₅-alkyl or C₁-C₅-halogenoalkyl comprising up to 9 halogen atoms that can be the same or different.
   - a heterocycle of formula (A²²) 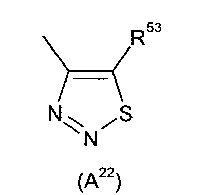
wherein :
R⁵³ represents a hydrogen atom ; a halogen atom ; C₁-C₅-alkyl or C₁-C₅-halogenoalkyl comprising up to 9 halogen atoms that can be the same or different.
   - a heterocycle of formula (A²³) 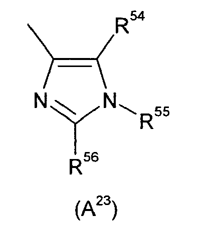
wherein :
R⁵⁴ and R⁵⁶ that can be the same or different represent a hydrogen atom ; a halogen atom ; C₁-C₅-alkyl or C₁-C₅-halogenoalkyl comprising up to 9 halogen atoms that can be the same or different ;
R⁵⁵ represents a hydrogen atom or C₁-C₅-alkyl ;
   - a heterocycle of formula (A²⁴) 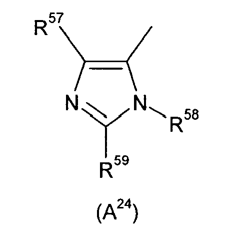
wherein :
R⁵⁷ and R⁵⁹ that can be the same or different represent a hydrogen atom ; a halogen atom ; C₁-C₅-alkyl or C₁-C₅-halogenoalkyl comprising up to 9 halogen atoms that can be the same or different ;
R⁵⁸ represents a hydrogen atom or C₁-C₅-alkyl ;
   - a heterocycle of formula (A²⁵) 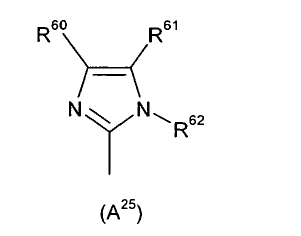
wherein :
R⁶⁰ and R⁶¹ that can be the same or different represent a hydrogen atom ; a halogen atom ; C₁-C₅-alkyl or C₁-C₅-halogenoalkyl comprising up to 9 halogen atoms that can be the same or different ;
R⁶² represents a hydrogen atom or C₁-C₅-alkyl ;
   - a heterocycle of formula (A²⁶) 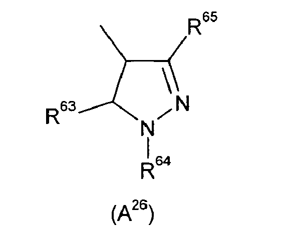
wherein :
R⁶⁵ represents a hydrogen atom ; a halogen atom ; C₁-C₅-alkyl ; C₃-C₅-cycloalkyl ; C₁-C₅-halogenoalkyl comprising up to 9 halogen atoms that can be the same or different ; C₁-C₅-alkoxy ; C₂-C₅-alkynyloxy or C₁-C₅-halogenoalkoxy comprising up to 9 halogen atoms that can be the same or different;
R⁶³ represents a hydrogen atom ; a halogen atom ; C₁-C₅-alkyl ; a cyano ; C₁-C₅-alkoxy ; C₁-C₅-alkylsulphanyl ; C₁-C₅-halogenoalkyl comprising up to 9 halogen atoms that can be the same or different ; C₁-C₅-halogenoalkoxy comprising up to 9 halogen atoms that can be the same or different; amino ; C₁-C₅-alkylamino or di(C₁-C₅-alkyl)amino ;
R⁶⁴ represents a hydrogen atom or C₁-C₅-alkyl.

More preferred compounds according to the invention are compounds of formula (I) wherein A is selected in the list consisting of A² ; A⁶ ; A¹⁰ and A¹³ as herein-defined.
Even more preferred compounds according to the invention are compounds of formula (I) wherein A represents A¹³_{,} wherein R³⁴ represents a C₁-C₅-alkyl, C₁-C₅-halogenoalkyl comprising up to 9 halogen atoms that can be the same or different ; or C₁-C₅-alkoxy ; wherein R³⁵ represents a hydrogen atom or a halogen atom and wherein R³⁶ represents a C₁-C₅-alkyl.

Other preferred compounds according to the invention are compounds of formula (I) wherein W represents a nitrogen atom or a carbon atom substituted by a hydrogen atom or a halogen atom.

Other preferred compounds according to the invention are compounds of formula (I) wherein n represents 0, 1 or 2.

Other preferred compounds according to the invention are compounds of formula (I) wherein X independently represents a halogen atom ; C₁-C₈-alkyl ; C₁-C₈-halogenoalkyl comprising up to 9 halogen atoms that can be the same or different ; tri(C₁-C₈-alkyl)silyl ; C₁-C₈-alkoxy or C₁-C₈-halogenoalkoxy comprising up to 9 halogen atoms that can be the same or different; or wherein two consecutive substituents X together with the phenyl ring form a substituted or non substituted 1,3-benzodioxolyl ; 1,2,3,4-tetrahydro-quinoxalinyl ; 3,4-dihydro-2H-1,4-benzoxazinyl ; 1,4-benzodioxanyl ; indanyl ; 2,3-dihydrobenzofuranyl ; or indolinyl.

Other more preferred compounds according to the invention are compounds of formula (I) wherein two consecutive substituents X together with the phenyl ring form a substituted or non substituted 1,3-benzodioxolyl ; 1,2,3,4-tetrahydro-quinoxalinyl ; 3,4-dihydro-2H-1,4-benzoxazinyl ; 1,4-benzodioxanyl ; indanyl ; 2,3-dihydrobenzofuranyl ; or indolinyl.

Other preferred compounds according to the invention are compounds of formula (I) wherein Z¹ and Z² independently represent a hydrogen atom, a halogen atom, a C₁-C₈-alkyl, a C₁-C₈-alkoxy or a C₁-C₈-halogenoalkyl having 1 to 5 halogen atoms.

Other preferred compounds according to the invention are compounds of formula (I) wherein Z³ and Z⁴ independently represent a hydrogen atom, a C₁-C₈-alkyl, a C₁-C₈-alkoxy or a C₁-C₈-halogenoalkyl having 1 to 5 halogen atoms.

Other preferred compounds according to the invention are compounds of formula (I) wherein Z⁵ represents a C₃-C₇ cycloalkyl substituted by up to 10 groups or atoms that can be the same or different and that can be selected in the list consisting of halogen atoms ; C₁-C₈-alkyl ; C₁-C₈-halogenoalkyl comprising up to 9 halogen atoms that can be the same or different ; C₁-C₈-alkoxy or C₁-C₈-halogenoalkoxy comprising up to 9 halogen atoms that can be the same or different;
More preferably Z⁵ represents a non-substituted C₃-C₇-cycloalkyl ;
Even more preferably Z⁵ represents cyclopropyl.

Other preferred compounds according to the invention are compounds of formula (I) wherein R independently represents a hydrogen atom ; halogen atom ; cyano ; C₁-C₈-alkylamino ; di-C₁-C₈-alkylamino ; tri(C₁-C₈-alkyl)silyl ; C₁-C₈-alkyl ; C₁-C₈-halogenoalkyl comprising up to 9 halogen atoms that can be the same or different ; C₁-C₈-alkoxy ; C₁-C₈-halogenoalkoxy comprising up to 9 halogen atoms that can be the same or different ; C₁-C₈-alkylsulfanyl ; amino, hydroxyl ; nitro ; C₁-C₈-alkoxycarbonyl ; or C₂-C₈-alkynyloxy.

The above mentioned preferences with regard to the substituents of the compounds according to the invention can be combined in various manners. These combinations of preferred features thus provide sub-classes of compounds according to the invention. Examples of such sub-classes of preferred compounds according to the invention can be combined:
- preferred features of A with preferred features of Z¹, Z², Z³, Z⁴, Z⁵, X, W, n and R;
- preferred features of Z¹ with preferred features of A, Z², Z³, Z⁴, Z⁵, X, W, n and R
- preferred features of Z² with preferred features of A, Z¹, Z³, Z⁴, Z⁵, X, W, n and R
- preferred features of Z³ with preferred features of A, Z¹, Z², Z⁴, Z⁵, X, W, n and R
- preferred features of Z⁴ with preferred features of A, Z¹, Z², Z³, Z⁵, X, W, n and R
- preferred features of Z⁵ with preferred features of A, Z¹, Z², Z³, Z⁴, X, W, n and R
- preferred features of X with preferred features of A, Z¹, Z², Z³, Z⁴, Z⁵, W, n and R
- preferred features of W with preferred features of A, Z¹, Z², Z³, Z⁴, Z⁵, X, n and R
- preferred features of n with preferred features of A, Z¹, Z², Z³, Z⁴, Z⁵, X, W and R
- preferred features of R with preferred features of A, Z¹, Z², Z³, Z⁴, Z⁵, X, W and n
   In these combinations of preferred features of the substituents of the compounds according to the invention, the said preferred features can also be selected among the more preferred features of each of A, Z¹, Z², Z³, Z⁴, Z⁵, X, W, n and R, so as to form most preferred subclasses of compounds according to the invention.
   According to a further aspect according to the invention, there is provided a process P1 for the preparation of a compound of formula (I) starting from a compound of formula (II) according to the following reaction scheme : 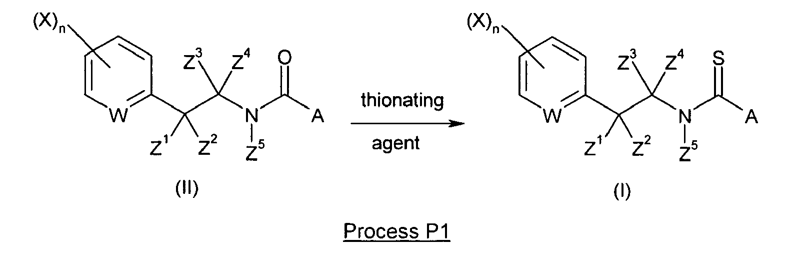
wherein X, n, W, Z¹, Z², Z³, Z⁴, Z⁵ and A are as herein-defined, in the optional presence of a catalytic or stoechiometric or more, quantity of a base such as an inorganic and organic base. Preference is given to using alkali metal carbonates, such as sodium carbonate, potassium carbonate, potassium bicarbonate, sodium bicarbonate ; heterocyclic aromatic bases, such as pyridine, picoline, lutidine, collidine ; and also tertiary amines, such as trimethylamine, triethylamine, tributylamine, N,N-dimethylaniline, N,N-dimethylaminopyridine or N-methylpiperidine.

Process P1 according to the invention can be performed in the presence of a thionating agent.

Starting amide derivatives of formula (II) are known or can be prepared by known processes such as for example in international patent applications WO-2005/085238 and WO-2007 /060164.

Suitable thionating agents for carrying out process P1 according to the invention can be sulphur (S), sulfhydric acid (H₂S), sodium sulfide (Na₂S), sodium hydrosulfide (NaHS), boron trisulfide (B₂S₃), bis (diethylaluminium) sulfide ((AlEt₂)₂S), ammonium sulfide ((NH₄)₂S), phosphorous pentasulfide (P₂S₅), Lawesson's reagent (2,4-bis(4-methoxyphenyl)-1,2,3,4-dithiadiphosphetane 2,4-disulfide) or a polymer-supported thionating reagent such as described in J.Chem.Soc. Perkin 1, (2001), 358*.*

The compound according to the present invention can be prepared according to the general processes of preparation described above. It will nevertheless be understood that, on the basis of his general knowledge and of available publications, the skilled worker will be able to adapt this method according to the specifics of each of the compounds, which it is desired to synthesize.

In a further aspect, the present invention also relates to a fungicide composition comprising an effective and non-phytotoxic amount of an active compound of formula (I). The expression "effective and non-phytotoxic amount" means an amount of composition according to the invention that is sufficient to control or destroy the fungi present or liable to appear on the cropsand that does not entail any appreciable symptom of phytotoxicity for the said crops. Such an amount can vary within a wide range depending on the fungus to be controlled, the type of crop, the climatic conditions and the compounds included in the fungicide composition according to the invention. This amount can be determined by systematic field trials that are within the capabilities of a person skilled in the art.

Thus, according to the invention, there is provided a fungicide composition comprising, as an active ingredient, an effective amount of a compound of formula (I) as herein defined and an agriculturally acceptable support, carrier or filler.

According to the invention, the term "support" denotes a natural or synthetic, organic or inorganic compound with that the active compound of formula (I) is combined or associated to make it easier to apply, notably to the parts of the plant. This support is thus generally inert and should be agriculturally acceptable. The support can be a solid or a liquid. Examples of suitable supports include clays, natural or synthetic silicates, silica, resins, waxes, solid fertilisers, water, alcohols, in particular butanol, organic solvents, mineral and plant oils and derivatives thereof. Mixtures of such supports can also be used.

The composition according to the invention can also comprise additional components. In particular, the composition can further comprise a surfactant. The surfactant can be an emulsifier, a dispersing agent or a wetting agent of ionic or non-ionic type or a mixture of such surfactants. Mention can be made, for example, of polyacrylic acid salts, lignosulphonic acid salts, phenolsulphonic or naphthalenesulphonic acid salts, polycondensates of ethylene oxide with fatty alcohols or with fatty acids or with fatty amines, substituted phenols (in particular alkylphenols or arylphenols), salts of sulphosuccinic acid esters, taurine derivatives (in particular alkyl taurates), phosphoric esters of polyoxyethylated alcohols or phenols, fatty acid esters of polyolsand derivatives of the above compounds containing sulphate, sulphonate and phosphate functions. The presence of at least one surfactant is generally essential when the active compound and/or the inert support are water-insoluble and when the vector agent for the application is water. Preferably, surfactant content can be comprised from 5% to 40% by weight of the composition.

Optionally, additional components can also be included, e.g. protective colloids, adhesives, thickeners, thixotropic agents, penetration agents, stabilisers, sequestering agents. More generally, the active compounds can be combined with any solid or liquid additive, that complies with the usual formulation techniques.

In general, the composition according to the invention can contain from 0.05 to 99% by weight of active compound, preferably 10 to 70% by weight.

Compositions according to the invention can be used in various forms such as aerosol dispenser, capsule suspension, cold fogging concentrate, dustable powder, emulsifiable concentrate, emulsion oil in water, emulsion water in oil, encapsulated granule, fine granule, flowable concentrate for seed treatment, gas (under pressure),gas generating product, granule, hot fogging concentrate, macrogranule, microgranule, oil dispersible powder, oil miscible flowable concentrate, oil miscible liquid, paste, plant rodlet, powder for dry seed treatment, seed coated with a pesticide, soluble concentrate, soluble powder, solution for seed treatment, suspension concentrate (flowable concentrate), ultra low volume (ULV) liquid, ultra low volume (ULV) suspension, water dispersible granules or tablets, water dispersible powder for slurry treatment, water soluble granules or tablets, water soluble powder for seed treatment and wettable powder. These compositions include not only compositions that are ready to be applied to the plant or seed to be treated by means of a suitable device, such as a spraying or dusting device, but also concentrated commercial compositions that must be diluted before application to the crop.

The compounds according to the invention can also be mixed with one or more insecticide, fungicide, bactericide, attractant, acaricide or pheromone active substance or other compounds with biological activity. The mixtures thus obtained have normally a broadened spectrum of activity. The mixtures with other fungicide compounds are particularly advantageous.

Examples of suitable fungicide mixing partners can be selected in the following lists:
(1) Inhibitors of the nucleic acid synthesis, for example benalaxyl, benalaxyl-M, bupirimate, clozylacon, dimethirimol, ethirimol, furalaxyl, hymexazol, metalaxyl, metalaxyl-M, ofurace, oxadixyl and oxolinic acid.
(2) Inhibitors of the mitosis and cell division, for example benomyl, carbendazim, chlorfenazole, diethofencarb, ethaboxam, fuberidazole, pencycuron, thiabendazole, thiophanate, thiophanate-methyl and zoxamide.
(3) Inhibitors of the respiration, for example diflumetorim as CI-respiration inhibitor; bixafen, boscalid, carboxin, fenfuram, flutolanil, fluopyram, furametpyr, furmecyclox, isopyrazam (9R-component), isopyrazam (9S-component), mepronil, oxycarboxin, penthiopyrad, sedaxane, thifluzamide as CII-respiration inhibitor; amisulbrom, azoxystrobin, cyazofamid, dimoxystrobin, enestroburin, famoxadone, fenamidone, fluoxastrobin, kresoxim-methyl, metominostrobin, orysastrobin, picoxystrobin, pyraclostrobin, pyribencarb, trifloxystrobin as CIII-respiration inhibitor.
(4) Compounds capable to act as an uncoupler, like for example binapacryl, dinocap, fluazinam and meptyldinocap.
(5) Inhibitors of the ATP production, for example fentin acetate, fentin chloride, fentin hydroxide, and silthiofam.
(6) Inhibitors of the amino acid and/or protein biosynthesis, for example andoprim, blasticidin-S, cyprodinil, kasugamycin, kasugamycin hydrochloride hydrate, mepanipyrim and pyrimethanil.
(7) Inhibitors of the signal transduction, for example fenpiclonil, fludioxonil and quinoxyfen.
(8) Inhibitors of the lipid and membrane synthesis, for example biphenyl, chlozolinate, edifenphos, etridiazole, iodocarb, iprobenfos, iprodione, isoprothiolane, procymidone, propamocarb, propamocarb hydrochloride, pyrazophos, tolclofos-methyl and vinclozolin.
(9) Inhibitors of the ergosterol biosynthesis, for example aldimorph, azaconazole, bitertanol, bromuconazole, cyproconazole, diclobutrazole, difenoconazole, diniconazole, diniconazole-M, dodemorph, dodemorph acetate, epoxiconazole, etaconazole, fenarimol, fenbuconazole, fenhexamid, fenpropidin, fenpropimorph, fluquinconazole, flurprimidol, flusilazole, flutriafol, furconazole, furconazole-cis, hexaconazole, imazalil, imazalil sulfate, imibenconazole, ipconazole, metconazole, myclobutanil, naftifine, nuarimol, oxpoconazole, paclobutrazol, pefurazoate, penconazole, piperalin, prochloraz, propiconazole, prothioconazole, pyributicarb, pyrifenox, quinconazole, simeconazole, spiroxamine, tebuconazole, terbinafine, tetraconazole, triadimefon, triadimenol, tridemorph, triflumizole, triforine, triticonazole, uniconazole, viniconazole and voriconazole.
(10) Inhibitors of the cell wall synthesis, for example benthiavalicarb, dimethomorph, flumorph, iprovalicarb, mandipropamid, polyoxins, polyoxorim, prothiocarb, validamycin A, and valiphenal.
(11) Inhibitors of the melanine biosynthesis, for example carpropamid, diclocymet, fenoxanil, phthalide, pyroquilon and tricyclazole.
(12) Compounds capable to induce a host defence, like for example acibenzolar-S-methyl, probenazole, and tiadinil.
(13) Compounds capable to have a multisite action, like for example bordeaux mixture, captafol, captan, chlorothalonil, copper naphthenate, copper oxide, copper oxychloride, copper preparations such as copper hydroxide, copper sulphate, dichlofluanid, dithianon, dodine, dodine free base, ferbam, fluorofolpet, folpet, guazatine, guazatine acetate, iminoctadine, iminoctadine albesilate, iminoctadine triacetate, mancopper, mancozeb, maneb, metiram, metiram zinc, oxine-copper, propamidine, propineb, sulphur and sulphur preparations including calcium polysulphide, thiram, tolylfluanid, zineb and ziram.
(14) Further compounds like for example 2,3-dibutyl-6-chlorothieno[2,3-d]pyrimidin-4(3H)-one, ethyl (2Z)-3-amino-2-cyano-3-phenylprop-2-enoate, N-[2-(1,3-dimethylbutyl)phenyl]-5-fluoro-1,3-dimethyl-1H-pyrazole-4-carboxamide, 3-(difluoromethyl)-1-methyl-N-(3',4',5'-trifluorobiphenyl-2-yl)-1H-pyrazole-4-carboxamide, 3-(difluoromethyl)-1-methyl-N-(2',4',5'-trifluorobiphenyl-2-yl)-1H-pyrazole-4-carboxamide,3-(difluoromethyl)-N-[4-fluoro-2-(1,1,2,3,3,3-hexafluoropropoxy)phenyl]-1-methyl-1H-pyrazole-4-carboxamide, (2E)-2-(2-{[6-(3-chloro-2-methylphenoxy)-5-fluoropyrimidin-4-yl]oxy}phenyl)-2-(methoxyimino)-N-methylethanamide, (2E)-2-{2-[({[(2E,3E)-4-(2,6-dichlorophenyl)but-3-en-2-ylidene]amino}oxy)methyl]phenyl}-2-(methoxyimino)-N-methylethanamide, 2-chloro-N-(1,1,3-trimethyl-2,3-dihydro-1H-inden-4-yl)pyridine-3-carboxamide, N-(3-ethyl-3,5,5-trimethylcyclohexyl)-3-(formylamino)-2-hydroxybenzamide, 5-methoxy-2-methyl-4-(2-{[({(1E)-1-[3-(trifluoromethyl)phenyl]ethylidene}amino)oxy]methyl}phenyl)-2,4-dihydro-3H-1,2,4-triazol-3-one, (2E)-2-(methoxyimino)-N-methyl-2-(2-{[({(1E)-1-[3-(trifluoromethyl)phenyl]ethylidene}amino)oxy]methyl}phenyl)ethanamide, (2E)-2-(methoxyimino)-N-methyl-2-{2-[(E)-({1-[3-(trifluoromethyl)phenyl]ethoxy}imino)methyl]phenyl}ethanamide, (2E)-2-{2-[({[(1E)-1-(3-{[(E)-1-fluoro-2-phenylethenyl]oxy}phenyl)ethylidene]amino}oxy)methyl]phenyl}-2-(methoxyimino)-N-methylethanamide, 1-(4-chlorophenyl)-2-(1H-1,2,4-triazol-1-yl)cycloheptanol, methyl 1-(2,2-dimethyl-2,3-dihydro-1H-inden-1-yl)-1H-imidazole-5-carboxylate, N-ethyl-N-methyl-N'-{2-methyl-5-(trifluoromethyl)-4-[3-(trimethylsilyl)propoxy]phenyl}imidoformamide, N'-{5-(difluoromethyl)-2-methyl-4-[3-(trimethylsilyl)propoxy]phenyl}-N-ethyl-N-methylimidoformamide, O-{1-[(4-methoxyphenoxy)methyl]-2,2-dimethylpropyl}1 H-imidazole-1-carbothioate, N-[2-(4-{[3-(4-chlorophenyl)prop-2-yn-1-yl]oxy}-3-methoxyphenyl)ethyl]-N²-(methylsulfonyl)valinamide, 5-chloro-7-(4-methylpiperidin-1-yl)-6-(2,4,6-trifluorophenyl)[1,2,4]triazolo[1,5-a]pyrimidine, 5-amino-1,3,4-thiadiazole-2-thiol, propamocarb-fosetyl, 1-[(4-methoxyphenoxy)methyl]-2,2-dimethylpropyl 1H-imidazole-1-carboxylate, 1-methyl-N-[2-(1,1,2,2-tetrafluoroethoxy)phenyl]-3-(trifluoromethyl)-1H-pyrazole-4-carboxamide, 2,3,5,6-tetrachloro-4-(methylsulfonyl)pyridine, 2-butoxy-6-iodo-3-propyl-4H-chromen-4-one, 2-phenylphenol and salts, 3-(difluoromethyl)-1-methyl-N-[2-(1,1,2,2-tetrafluoroethoxy)phenyl]-1H-pyrazole-4-carboxamide, 3,4,5-trichloropyridine-2,6-dicarbonitrile, 3-[5-(4-chlorophenyl)-2,3-dimethylisoxazolidin-3-yl]pyridine, 3-chloro-5-(4-chlorophenyl)-4-(2,6-difluorophenyl)-6-methylpyridazine, 4-(4-chlorophenyl)-5-(2,6-difluorophenyl)-3,6-dimethylpyridazine, quinolin-8-ol, quinolin-8-ol sulfate (2:1) (salt), 5-methyl-6-octyl-3,7-dihydro[1,2,4]triazolo[1,5-a]pyrimidin-7-amine, 5-methoxymethyl-6-octyl-3,7-dihydro[1,2,4]triazolo[1,5-a]pyrimidin-7-amine, 5-ethyl-6-octyl-3,7-dihydro[1,2,4]triazolo[1,5-a]pyrimidin-7-amine, benthiazole, bethoxazin, capsimycin, carvone, chinomethionat, chloroneb, cufraneb, cyflufenamid, cymoxanil, cyprosulfamide, dazomet, debacarb, dichlorophen, diclomezine, dicloran, difenzoquat, difenzoquat methylsulphate, diphenylamine, ecomate, ferimzone, flumetover, fluopicolide, fluoroimide, flusulfamide, flutianil, fosetyl-aluminium, fosetyl-calcium, fosetyl-sodium, hexachlorobenzene, irumamycin, isotianil, methasulfocarb, methyl (2E)-2-{2-[({cyclopropyl[(4-methoxyphenyl)imino]methyl}thio)methyl]phenyl}-3-methoxyacrylate, methyl isothiocyanate, metrafenone, (5-chloro-2-methoxy-4-methylpyridin-3-yl)(2,3,4-trimethoxy-6-methylphenyl)methanone, mildiomycin, tolnifanide, N-(4-chlorobenzyl)-3-[3-methoxy-4-(prop-2-yn-1-yloxy)phenyl]propanamide, N-[(4-chlorophenyl)(cyano)methyl]-3-[3-methoxy-4-(prop-2-yn-1-yloxy)phenyl]propanamide, N-[(5-bromo-3-chloropyridin-2-yl)methyl]-2,4-dichloropyridine-3-carboxamide, N-[1-(5-bromo-3-chloropyridin-2-yl)ethyl]-2,4-dichloropyridine-3-carboxamide, N-[1-(5-bromo-3-chloropyridin-2-yl)ethyl]-2-fluoro-4-iodopyridine-3-carboxamide, N-{(Z)-[(cyclopropylmethoxy)imino][6-(difluoromethoxy)-2,3-difluorophenyl]methyll-2-phenylacetamide, N-{(E)-[(cyclopropylmethoxy)imino][6-(difluoromethoxy)-2,3-difluorophenyl]methyl}-2-phenylacetamide, natamycin, nickel dimethyldithiocarbamate, nitrothal-isopropyl, octhilinone, oxamocarb, oxyfenthiin, pentachlorophenol and salts, phenazine-1-carboxylic acid, phenothrin, phosphorous acid and its salts, propamocarb fosetylate, propanosine-sodium, proquinazid, pyrrolnitrine, quintozene, S-prop-2-en-1-yl 5-amino-2-(1-methylethyl)-4-(2-methylphenyl)-3-oxo-2,3-dihydro-1 H-pyrazole-1-carbothioate, tecloftalam, tecnazene, triazoxide, trichlamide, 5-chloro-N'-phenyl-N'-prop-2-yn-1-ylthiophene-2-sulfonohydrazide, zarilamid, N-methyl-2-(1-{[5-methyl-3-(trifluoromethyl)-1H-pyrazol-1-yl]acetyl}piperidin-4-yl)-N-[(1R)-1,2,3,4-tetrahydronaphthalen-1-yl]-1 ,3-thiazole-4-carboxamide, N-methyl-2-(1-{[5-methyl-3-(trifluoromethyl)-1H-pyrazol-1-yl]acetyl}piperidin-4-yl)-N-(1,2,3,4-tetrahydronaphthalen-1-yl)-1,3-thiazole-4-carboxamide, 3-(difluoromethyl)-N-[4-fluoro-2-(1,1,2,3,3,3-hexafluoropropoxy)phenyl]-1-methyl-1H-pyrazole-4-carboxamide and pentyl {6-[({[(1-methyl-1H-tetrazol-5-yl)(phenyl)methylidene]amino}oxy)methyl]pyridin-2-yl}carbamate.

The composition according to the invention comprising a mixture of a compound of formula (I) with a bactericide compound can also be particularly advantageous. Examples of suitable bactericide mixing partners can be selected in the following list: bronopol, dichlorophen, nitrapyrin, nickel dimethyldithiocarbamate, kasugamycin, octhilinone, furancarboxylic acid, oxytetracycline, probenazole, streptomycin, tecloftalam, copper sulphate and other copper preparations.

The compounds of formula (I) and the fungicide composition according to the invention can be used to curatively or preventively control the phytopathogenic fungi of plants or crops.

Thus, according to a further aspect of the invention, there is provided a method for curatively or preventively controlling the phytopathogenic fungi of plants or crops characterised in that a compound of formula (I) or a fungicide composition according to the invention is applied to the seed, the plant or to the fruit of the plant or to the soil wherein the plant is growing or wherein it is desired to grow.
The method of treatment according to the invention can also be useful to treat propagation material such as tubers or rhizomes, but also seeds, seedlings or seedlings pricking out and plants or plants pricking out. This method of treatment can also be useful to treat roots. The method of treatment according to the invention can also be useful to treat the overground parts of the plant such as trunks, stems or stalks, leaves, flowers and fruit of the concerned plant.

Among the plants that can be protected by the method according to the invention, mention can be made of cotton; flax; vine; fruit or vegetable crops such as *Rosaceae sp.* (for instance pip fruit such as apples and pears, but also stone fruit such as apricots, almonds and peaches), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for instance banana trees and plantins), *Rubiaceae sp., Theaceae sp., Sterculiceae sp., Rutaceae sp.* (for instance lemons oranges and grapefruit); *Solanaceae sp.* (for instance tomatoes), *Liliaceae sp., Asteraceae sp.* (for instance lettuces), *Umbelliferae sp., Cruciferae sp., Chenopodiaceae sp., Cucurbitaceae sp., Papilionaceae sp.* (for instance peas), *Rosaceae sp.* (for instance strawberries); major crops such as *Graminae sp.* (for instance maize, lawn or cereals such as wheat, rye, rice, barley and triticale), *Asteraceae sp.* (for instance sunflower), *Cruciferae sp.* (for instance colza), *Fabacae sp.* (for instance peanuts), *Papilionaceae sp.* (for instance soybean), *Solanaceae sp.* (for instance potatoes), *Chenopodiaceae sp.* (for instance beetroots), *Elaeis sp.* (for instance oil palm); horticultural and forest crops; as well as genetically modified homologues of these crops.

The method of treatment according to the invention can be used in the treatment of genetically modified organisms (GMOs), e.g. plants or seeds. Genetically modified plants (or transgenic plants) are plants in which a heterologous gene has been stably integrated into the genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, co suppression technology or RNA interference - RNAi - technology). A heterologous gene that is located in the genome is also called a transgene. A transgene that is defined by its particular location in the plant genome is called a transformation or transgenic event.

Depending on the plant species or plant cultivars, their location and growth conditions (soils, climate, vegetation period, diet), the treatment according to the invention may also result in superadditive ("synergistic") effects. Thus, for example, reduced application rates and/or a widening of the activity spectrum and/or an increase in the activity of the active compounds and compositions which can be used according to the invention, better plant growth, increased tolerance to high or low temperatures, increased tolerance to drought or to water or soil salt content, increased flowering performance, easier harvesting, accelerated maturation, higher harvest yields, bigger fruits, larger plant height, greener leaf color, earlier flowering, higher quality and/or a higher nutritional value of the harvested products, higher sugar concentration within the fruits, better storage stability and/or processability of the harvested products are possible, which exceed the effects which were actually to be expected.

At certain application rates, the active compound combinations according to the invention may also have a strengthening effect in plants. Accordingly, they are also suitable for mobilizing the defense system of the plant against attack by unwanted phytopathogenic fungi and/ or microorganisms and/or viruses. This may, if appropriate, be one of the reasons of the enhanced activity of the combinations according to the invention, for example against fungi. Plant-strengthening (resistance-inducing) substances are to be understood as meaning, in the present context, those substances or combinations of substances which are capable of stimulating the defense system of plants in such a way that, when subsequently inoculated with unwanted phytopathogenic fungi and/ or microorganisms and/or viruses, the treated plants display a substantial degree of resistance to these unwanted phytopathogenic fungi and/ or microorganisms and/or viruses. In the present case, unwanted phytopathogenic fungi and/ or microorganisms and/or viruses are to be understood as meaning phytopathogenic fungi, bacteria and viruses. Thus, the substances according to the invention can be employed for protecting plants against attack by the abovementioned pathogens within a certain period of time after the treatment. The period of time within which protection is effected generally extends from 1 to 10 days, preferably 1 to 7 days, after the treatment of the plants with the active compounds.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozon exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed composition, such as carbohydrate content, protein content, oil content and composition, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stress factors. Such plants are typically made by crossing an inbred male-sterile parent line (the female parent) with another inbred male-fertile parent line (the male parent). Hybrid seed is typically harvested from the male sterile plants and sold to growers. Male sterile plants can sometimes (e.g. in corn) be produced by detasseling, i.e. the mechanical removal of the male reproductive organs (or males flowers) but, more typically, male sterility is the result of genetic determinants in the plant genome. In that case, and especially when seed is the desired product to be harvested from the hybrid plants it is typically useful to ensure that male fertility in the hybrid plants is fully restored. This can be accomplished by ensuring that the male parents have appropriate fertility restorer genes which are capable of restoring the male fertility in hybrid plants that contain the genetic determinants responsible for male-sterility. Genetic determinants for male sterility may be located in the cytoplasm. Examples of cytoplasmic male sterility (CMS) were for instance described in Brassica species (W0 1992/005251, WO 1995/009910, WO 1998/27806, WO 2005/002324, WO 2006/021972 and US 6,229,072). However, genetic determinants for male sterility can also be located in the nuclear genome. Male sterile plants can also be obtained by plant biotechnology methods such as genetic engineering. A particularly useful means of obtaining male-sterile plants is described in WO 1989/10396 in which, for example, a ribonuclease such as barnase is selectively expressed in the tapetum cells in the stamens. Fertility can then be restored by expression in the tapetum cells of a ribonuclease inhibitor such as barstar (e.g. WO 1991/002069).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.
Herbicide-tolerant plants are for example glyphosate-tolerant plants, i.e. plants made tolerant to the herbicide glyphosate or salts thereof. Plants can be made tolerant to glyphosate through different means. For example, glyphosate-tolerant plants can be obtained by transforming the plant with a gene encoding the enzyme 5-enolpyruvylshikimate-3-phosphate synthase (EPSPS). Examples of such EPSPS genes are the AroA gene (mutant CT7) of the bacterium *Salmonella typhimurium* (Comai et al., Science (1983), 221, 370-371), the CP4 gene of the bacterium *Agrobacterium sp.* (Barry et al., Curr. Topics Plant Physiol. (1992), 7, 139-145), the genes encoding a Petunia EPSPS (Shah et al., Science (1986), 233, 478-481), a Tomato EPSPS (Gasser et al., J. Biol. Chem. (1988),263, 4280-4289), or an Eleusine EPSPS (WO 2001/66704). It can also be a mutated EPSPS as described in for example EP-A 0837944, WO 2000/066746, WO 2000/066747 or WO 2002/026995. Glyphosate-tolerant plants can also be obtained by expressing a gene that encodes a glyphosate oxido-reductase enzyme as described in US 5,776,760 and US 5,463,175. Glyphosate-tolerant plants can also be obtained by expressing a gene that encodes a glyphosate acetyl transferase enzyme as described in for example WO 2002/036782, WO 2003/092360, WO 2005/012515 and WO 2007/024782. Glyphosate-tolerant plants can also be obtained by selecting plants containing naturally-occurring mutations of the abovementioned genes, as described in for example WO 2001/024615 or WO 2003/013226. Other herbicide resistant plants are for example plants that are made tolerant to herbicides inhibiting the enzyme glutamine synthase, such as bialaphos, phosphinothricin or glufosinate. Such plants can be obtained by expressing an enzyme detoxifying the herbicide or a mutant glutamine synthase enzyme that is resistant to inhibition. One such efficient detoxifying enzyme is an enzyme encoding a phosphinothricin acetyltransferase (such as the bar or pat protein from Streptomyces species). Plants expressing an exogenous phosphinothricin acetyltransferase are for example described in US 5,561,236; US 5,648,477; US 5,646,024; US 5,273,894; US 5,637,489; US 5,276,268; US 5,739,082; US 5,908,810 and US 7,112,665.
Further herbicide-tolerant plants are also plants that are made tolerant to the herbicides inhibiting the enzyme hydroxyphenylpyruvatedioxygenase (HPPD).
Hydroxyphenylpyruvatedioxygenases are enzymes that catalyze the reaction in which parahydroxyphenylpyruvate (HPP) is transformed into homogentisate. Plants tolerant to HPPD-inhibitors can be transformed with a gene encoding a naturally-occurring resistant HPPD enzyme, or a gene encoding a mutated HPPD enzyme as described in WO 1996/038567, WO 1999/024585 and WO 1999/024586. Tolerance to HPPD-inhibitors can also be obtained by transforming plants with genes encoding certain enzymes enabling the formation of homogentisate despite the inhibition of the native HPPD enzyme by the HPPD-inhibitor.
Such plants and genes are described in WO 1999/034008 and WO 2002/36787. Tolerance of plants to HPPD inhibitors can also be improved by transforming plants with a gene encoding an enzyme prephenate dehydrogenase in addition to a gene encoding an HPPD-tolerant enzyme, as described in WO 2004/024928.
Still further herbicide resistant plants are plants that are made tolerant to acetolactate synthase (ALS) inhibitors. Known ALS-inhibitors include, for example, sulfonylurea, imidazolinone, triazolopyrimidines, pyrimidinyloxy(thio)benzoates, and/or sulfonylaminocarbonyltriazolinone herbicides. Different mutations in the ALS enzyme (also known as acetohydroxyacid synthase, AHAS) are known to confer tolerance to different herbicides and groups of herbicides, as described for example in Tranel and Wright, Weed Science (2002), 50, 700-712, but also, in US 5,605,011, US 5,378,824, US 5,141,870, and US 5,013,659. The production of sulfonylurea-tolerant plants and imidazolinone-tolerant plants is described in US 5,605,011; US 5,013,659; US 5,141,870; US 5,767,361; US 5,731,180; US 5,304,732; US 4,761,373; US 5,331,107; US 5,928,937; and US 5,378,824; and international publication WO 1996/033270. Other imidazolinone-tolerant plants are also described in for example WO 2004/040012, WO 2004/106529, WO 2005/020673, WO 2005/093093, WO 2006/007373, WO 2006/015376, WO 2006/024351, and WO 2006/060634. Further sulfonylurea- and imidazolinone-tolerant plants are also described in for example WO 2007/024782.
Other plants tolerant to imidazolinone and/or sulfonylurea can be obtained by induced mutagenesis, selection in cell cultures in the presence of the herbicide or mutation breeding as described for example for soybeans in US 5,084,082, for rice in WO 1997/41218, for sugar beet in US 5,773,702 and WO 1999/057965 , for lettuce in US 5,198,599, or for sunflower in WO 2001/065922.
Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.
An "insect-resistant transgenic plant", as used herein, includes any plant containing at least one transgene comprising a coding sequence encoding:
1) an insecticidal crystal protein from *Bacillus thuringiensis* or an insecticidal portion thereof, such as the insecticidal crystal proteins listed by Crickmore et al., Microbiology and Molecular Biology Reviews (1998), 62, 807-813, updated by Crickmore et al. (2005) at the Bacillus thuringiensis toxin nomenclature, online at: http://www.lifesci.sussex.ac.uk/Home/Neil_Crickmore/Bt/), or insecticidal portions thereof, e.g., proteins of the Cry protein classes Cry1Ab, Cry1Ac, Cry1F, Cry2Ab, Cry3Aa, or Cry3Bb or insecticidal portions thereof; or
2) a crystal protein from *Bacillus thuringiensis* or a portion thereof which is insecticidal in the presence of a second other crystal protein from *Bacillus thuringiensis* or a portion thereof, such as the binary toxin made up of the Cry34 and Cry35 crystal proteins (Moellenbeck et al., Nat. Biotechnol. (2001), 19, 668-72; Schnepf et al., Applied Environm. Microbiol. (2006), 71, 1765-1774); or
3) a hybrid insecticidal protein comprising parts of different insecticidal crystal proteins from *Bacillus thuringiensis,* such as a hybrid of the proteins of 1) above or a hybrid of the proteins of 2) above, e.g., the Cry1A.105 protein produced by corn event MON98034 (WO 2007/027777); or
4) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation, such as the Cry3Bb1 protein in corn events MON863 or MON88017, or the Cry3A protein in corn event MIR604;
5) an insecticidal secreted protein from *Bacillus thuringiensis* or *Bacillus cereus,* or an insecticidal portion thereof, such as the vegetative insecticidal (VIP) proteins listed at:
   http://www.lifesci.sussex.ac.uk/home/Neil_Crickmore/Bt/vip.html, e.g., proteins from the VIP3Aa protein class; or
6) a secreted protein from *Bacillus thuringiensis* or *Bacillus cereus* which is insecticidal in the presence of a second secreted protein from *Bacillus thuringiensis* or *B. cereus,* such as the binary toxin made up of the VIP1A and VIP2A proteins (WO 1994/21795); or
7) a hybrid insecticidal protein comprising parts from different secreted proteins from *Bacillus thuringiensis* or *Bacillus cereus,* such as a hybrid of the proteins in 1) above or a hybrid of the proteins in 2) above; or
8) a protein of any one of 1) to 3) above wherein some, particularly 1 to 10, amino acids have been replaced by another amino acid to obtain a higher insecticidal activity to a target insect species, and/or to expand the range of target insect species affected, and/or because of changes introduced into the encoding DNA during cloning or transformation (while still encoding an insecticidal protein), such as the VIP3Aa protein in cotton event COT102.

Of course, an insect-resistant transgenic plant, as used herein, also includes any plant comprising a combination of genes encoding the proteins of any one of the above classes 1 to 8. In one embodiment, an insect-resistant plant contains more than one transgene encoding a protein of any one of the above classes 1 to 8, to expand the range of target insect species affected when using different proteins directed at different target insect species, or to delay insect resistance development to the plants by using different proteins insecticidal to the same target insect species but having a different mode of action, such as binding to different receptor binding sites in the insect.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance. Particularly useful stress tolerance plants include:
a. plants which contain a transgene capable of reducing the expression and/or the activity of poly(ADP-ribose)polymerase (PARP) gene in the plant cells or plants as described in WO 2000/004173 or WO2006/045633 or PCT/EP07/004142.
b. plants which contain a stress tolerance enhancing transgene capable of reducing the expression and/or the activity of the PARG encoding genes of the plants or plants cells, as described e.g. in WO 2004/090140.
c. plants which contain a stress tolerance enhancing transgene coding for a plant-functional enzyme of the nicotinamide adenine dinucleotide salvage synthesis pathway including nicotinamidase, nicotinate phosphoribosyltransferase, nicotinic acid mononucleotide adenyl transferase, nicotinamide adenine dinucleotide synthetase or nicotine amide phosphoribosyltransferase as described e.g. in WO2006/032469 or WO 2006/133827 or PCT/EP07/002433.

Plants or plant cultivars (obtained by plant biotechnology methods such as genetic engineering) which may also be treated according to the invention show altered quantity, quality and/or storage-stability of the harvested product and/or altered properties of specific ingredients of the harvested product such as :
1) transgenic plants which synthesize a modified starch, which in its physical-chemical characteristics, in particular the amylose content or the amylose/amylopectin ratio, the degree of branching, the average chain length, the side chain distribution, the viscosity behaviour, the gelling strength, the starch grain size and/or the starch grain morphology, is changed in comparison with the synthesised starch in wild type plant cells or plants, so that this is better suited for special applications. Said transgenic plants synthesizing a modified starch are disclosed, for example, in EP 0571427, WO 1995/004826, EP 0719338, WO 1996/15248, WO 1996/19581, WO 1996/27674, WO 1997/11188, WO 1997/26362, WO 1997/32985, WO 1997/42328, WO 1997/44472, WO 1997/45545, WO 1998/27212, WO 1998/40503, WO99/58688, WO 1999/58690, WO 1999/58654, WO 2000/008184, WO 2000/008185, WO 2000/008175, WO 2000/28052, WO 2000/77229, WO 2001/12782, WO 2001/12826, WO 2002/101059, WO 2003/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 2000/22140, WO 2006/063862, WO 2006/072603, WO 2002/034923, EP 06090134.5, EP 06090228.5, EP 06090227.7, EP 07090007.1, EP 07090009.7, WO 2001/14569, WO 2002/79410, WO 2003/33540, WO 2004/078983, WO 2001/19975, WO 1995/26407, WO 1996/34968, WO 1998/20145, WO 1999/12950, WO 1999/66050, WO 1999/53072, US 6,734,341, WO 2000/11192, WO 1998/22604, WO 1998/32326, WO 2001/98509, WO 2001/98509, WO 2005/002359, US 5,824,790, US 6,013,861, WO 1994/004693, WO 1994/009144, WO 1994/11520, WO 1995/35026, WO 1997/20936.
2) transgenic plants which synthesize non starch carbohydrate polymers or which synthesize non starch carbohydrate polymers with altered properties in comparison to wild type plants without genetic modification. Examples are plants producing polyfructose, especially of the inulin and levan-type, as disclosed in EP 0663956, WO 1996/001904, WO 1996/021023, WO 1998/039460, and WO 1999/024593, plants producing alpha 1,4 glucans as disclosed in WO 1995/031553, US 2002/031826, US 6,284,479, US 5,712,107, WO 1997/047806, WO 1997/047807, WO 1997/047808 and WO 2000/014249, plants producing alpha-1,6 branched alpha-1,4-glucans, as disclosed in WO 2000/73422, plants producing alternan, as disclosed in WO 2000/047727, EP 06077301.7, US 5,908,975 and EP 0728213,
3) transgenic plants which produce hyaluronan, as for example disclosed in WO 2006/032538, WO 2007/039314, WO 2007/039315, WO 2007/039316, JP 2006/304779, and WO 2005/012529.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics and include:
a) Plants, such as cotton plants, containing an altered form of cellulose synthase genes as described in WO 1998/000549
b) Plants, such as cotton plants, containing an altered form of rsw2 or rsw3 homologous nucleic acids as described in WO2004/053219
c) Plants, such as cotton plants, with increased expression of sucrose phosphate synthase as described in WO 2001/017333
d) Plants, such as cotton plants, with increased expression of sucrose synthase as described in WO02/4548
e) Plants, such as cotton plants, wherein the timing of the plasmodesmatal gating at the basis of the fiber cell is altered, e.g. through downregulation of fiberselective β 1,3-glucanase as described in WO2005/017157
f) Plants, such as cotton plants, having fibers with altered reactivity, e.g. through the expression of N-acteylglucosaminetransferase gene including nodC and chitinsynthase genes as described in WO2006/136351

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation or by selection of plants contain a mutation imparting such altered oil characteristics and include:
a) Plants, such as oilseed rape plants, producing oil having a high oleic acid content as described e.g. in US 5,969,169, US 5,840,946 or US 6,323,392 or US 6,063,947
b) Plants such as oilseed rape plants, producing oil having a low linolenic acid content as described in US 6,270828, US 6,169,190 or US 5,965,755
c) Plant such as oilseed rape plants, producing oil having a low level of saturated fatty acids as described e.g. in US 5,434,283

Particularly useful transgenic plants which may be treated according to the invention are plants which comprise one or more genes which encode one or more toxins, such as the following which are sold under the trade names YIELD GARD® (for example maize, cotton, soya beans), KnockOut® (for example maize), BiteGard® (for example maize), Bt-Xtra® (for example maize), StarLink® (for example maize), Bollgard® (cotton), Nucotn® (cotton), Nucotn 33B®(cotton), NatureGard® (for example maize), Protecta® and NewLeaf® (potato). Examples of herbicide-tolerant plants which may be mentioned are maize varieties, cotton varieties and soya bean varieties which are sold under the trade names Roundup Ready® (tolerance to glyphosate, for example maize, cotton, soya bean), Liberty Link® (tolerance to phosphinotricin, for example oilseed rape), IMI® (tolerance to imidazolinones) and STS® (tolerance to sulphonylureas, for example maize). Herbicide-resistant plants (plants bred in a conventional manner for herbicide tolerance) which may be mentioned include the varieties sold under the name Clearfield® (for example maize).

Particularly useful transgenic plants which may be treated according to the invention are plants containing transformation events, or combination of transformation events, that are listed for example in the databases from various national or regional regulatory agencies (see for example http://gmoinfo.jrc.it/gmp_browse.aspx and
http://www.agbios.com/dbase.php).

Among the diseases of plants or crops that can be controlled by the method according to the invention, mention can be made of :
Powdery mildew diseases such as :
   Blumeria diseases, caused for example by *Blumeria graminis* ;
   Podosphaera diseases, caused for example by *Podosphaera leucotricha* ;
   Sphaerotheca diseases, caused for example by *Sphaerotheca fuliginea* ;
   Uncinula diseases, caused for example by *Uncinula necator*;
Rust diseases such as :
   Gymnosporangium diseases, caused for example by *Gymnosporangium sabinae* ;
   Hemileia diseases, caused for example by *Hemileia vastatrix* ;
   Phakopsora diseases, caused for example by *Phakopsora pachyrhizi* or *Phakopsora meibomiae* ;
   Puccinia diseases, caused for example by *Puccinia recondite, Puccinia graminis or Puccinia striiformis*;
   Uromyces diseases, caused for example by *Uromyces appendiculatus* ;
Oomycete diseases such as :
   Albugo diseases caused for example by *Albugo candida*;
   Bremia diseases, caused for example by *Bremia lactucae* ;
   Peronospora diseases, caused for example by *Peronospora pisi* or *P. brassicae* ;
   Phytophthora diseases, caused for example by *Phytophthora infestans* ;
   Plasmopara diseases, caused for example by Plasmopara *viticola* ;
   Pseudoperonospora diseases, caused for example by *Pseudoperonospora humuli* or
*Pseudoperonospora cubensis ;*
   Pythium diseases, caused for example by *Pythium ultimum* ;
Leafspot, leaf blotch and leaf blight diseases such as :
   Alternaria diseases, caused for example by *Alternaria solani* ;
   Cercospora diseases, caused for example by *Cercospora beticola* ;
   Cladiosporum diseases, caused for example by *Cladiosporium cucumerinum* ;
   Cochliobolus diseases, caused for example by *Cochliobolus sativus* (Conidiaform: Drechslera, Syn: Helminthosporium) or *Cochliobolus miyabeanus* ;
   Colletotrichum diseases, caused for example by *Colletottichum lindemuthanium* ;
   Cycloconium diseases, caused for example by *Cycloconium oleaginum* ;
   Diaporthe diseases, caused for example by *Diaporthe citri* ;
   Elsinoe diseases, caused for example by *Elsinoe fawcettii* ;
   Gloeosporium diseases, caused for example by *Gloeosporium laeticolor* ;
   Glomerella diseases, caused for example by *Glomerella cingulata ;*
   Guignardia diseases, caused for example by *Guignardia bidwelli* ;
   Leptosphaeria diseases, caused for example by *Leptosphaeria maculans* ; *Leptosphaeria nodorum* ;
   Magnaporthe diseases, caused for example by *Magnaporthe grisea* ;
   Mycosphaerella diseases, caused for example by *Mycosphaerella graminicola* ;
Mycosphaerella arachidicola ; *Mycosphaerella fijiensis* ;
   Phaeosphaeria diseases, caused for example by *Phaeosphaeria nodorum* ;
   Pyrenophora diseases, caused for example by *Pyrenophora teres,* or *Pyrenophora tritici repentis*;
   Ramularia diseases, caused for example by *Ramularia collo-cygni* , or *Ramularia areola*;
   Rhynchosporium diseases, caused for example by *Rhynchosporium secalis* ;
   Septoria diseases, caused for example by *Septoria apii* or *Septoria lycopercisi* ;
   Typhula diseases, caused for example by *Typhula incarnata* ;
   Venturia diseases, caused for example by *Venturia inaequalis* ;
Root, Sheath and stem diseases such as :
   Corticium diseases, caused for example by *Corticium graminearum* ;
   Fusarium diseases, caused for example by *Fusarium oxysporum* ;
   Gaeumannomyces diseases, caused for example by *Gaeumannomyces graminis* ;
   Rhizoctonia diseases, caused for example by *Rhizoctonia solani* ;
   Sarocladium diseases caused for example by *Sarocladium oryzae*;
   Sclerotium diseases caused for example by *Sclerotium oryzae*;
   Tapesia diseases, caused for example by *Tapesia acuformis* ;
   Thielaviopsis diseases, caused for example by *Thielaviopsis basicola* ;
Ear and panicle diseases such as :
   Alternaria diseases, caused for example by *Alternaria spp*. ;
   Aspergillus diseases, caused for example by *Aspergillus flavus* ;
   Cladosporium diseases, caused for example by *Cladosporium spp*. ;
   Claviceps diseases, caused for example by *Claviceps purpurea* ;
   Fusarium diseases, caused for example by *Fusarium culmorum* ;
   Gibberella diseases, caused for example by *Gibberella zeae* ;
   Monographella diseases, caused for example by *Monographella nivalis* ;
Smut and bunt diseases such as :
   Sphacelotheca diseases, caused for example by *Sphacelotheca reiliana* ;
   Tilletia diseases, caused for example by *Tilletia caries* ;
   Urocystis diseases, caused for example by *Urocystis occulta* ;
   Ustilago diseases, caused for example by *Ustilago nuda* ;
Fruit rot and mould diseases such as :
   Aspergillus diseases, caused for example by *Aspergillus flavus* ;
   Botrytis diseases, caused for example by *Botrytis cinerea* ;
   Penicillium diseases, caused for example by *Penicillium expansum* ;
   Rhizopus diseases caused by example by *Rhizopus stolonifer* Sclerotinia diseases, caused for example by *Sclerotinia sclerotiorum* ;
   Verticilium diseases, caused for example by *Verticilium alboatrum* ;
Seed and soilborne decay, mould, wilt, rot and damping-off diseases :
   Alternaria diseases, caused for example by *Alternaria brassicicola*
   Aphanomyces diseases, caused for example by *Aphanomyces euteiches*
   Ascochyta diseases, caused for example by *Ascochyta lentis*
   Aspergillus diseases, caused for example by *Aspergillus flavus*
   Cladosporium diseases, caused for example by *Cladosporium herbarum*
   Cochliobolus diseases, caused for example by *Cochliobolus sativus* (Conidiaform: *Drechslera, Bipolaris Syn: Helminthosporium*);
   Colletotrichum diseases, caused for example by *Colletotrichum coccodes*;
   Fusarium diseases, caused for example by *Fusarium culmorum*;
   Gibberella diseases, caused for example by *Gibberella zeae*;
   Macrophomina diseases, caused for example by *Macrophomina* phaseolina
   Monographella diseases, caused for example by *Monographella nivalis*;
   Penicillium diseases, caused for example by *Penicillium expansum* Phoma diseases, caused for example by *Phoma lingam* Phomopsis diseases, caused for example by *Phomopsis sojae*;
   Phytophthora diseases, caused for example by Phytophthora cactorum;
   Pyrenophora diseases, caused for example by *Pyrenophora graminea*
   Pyricularia diseases, caused for example by *Pyricularia oryzae*;
   Pythium diseases, caused for example by *Pythium ultimum*;
   Rhizoctonia diseases, caused for example by *Rhizoctonia solani*;
   Rhizopus diseases, caused for example by *Rhizopus oryzae*
   Sclerotium diseases, caused for example by *Sclerotium rolfsii*;
   Septoria diseases, caused for example by *Septoria nodorum*;
   Typhula diseases, caused for example by *Typhula incarnata*;
   Verticillium diseases, caused for example by *Verticillium dahliae* ;
Canker, broom and dieback diseases such as :
   Nectria diseases, caused for example by *Nectria galligena* ;
Blight diseases such as :
   Monilinia diseases, caused for example by *Monilinia laxa* ;
Leaf blister or leaf curl diseases such as :
   Exobasidium diseases caused for example by *Exobasidium vexans*
   Taphrina diseases, caused for example by *Taphrina deformans* ;
Decline diseases of wooden plants such as :
   Esca diseases, caused for example by *Phaemoniella clamydospora* ;
   Eutypa dyeback, caused for example by *Eutypa lata* ;
   Ganoderma diseases caused for example by *Ganoderma boninense*;
   Rigidoporus diseases caused for example by *Rigidoporus lignosus*

Diseases of Flowers and Seeds such as
Botrytis diseases caused for example by *Botrytis cinerea*;

Diseases of Tubers such as
Rhizoctonia diseases caused for example by *Rhizoctonia solani*;
Helminthosporium diseases caused for example by *Helminthosporium solani*;

Club root diseases such as
Plasmodiophora diseases, cause for example by *Plamodiophora* brassicae.

Diseases caused by Bacterial Organisms such as
Xanthomonas species for example Xanthomonas *campestris* pv. oryzae;
Pseudomonas species for example *Pseudomonas syringae* pv. lachrymans;
Erwinia species for example *Erwinia amylovora.*

The fungicide composition according to the invention can also be used against fungal diseases liable to grow on or inside timber. The term "timber" means all types of species of woodand all types of working of this wood intended for construction, for example solid wood, high-density wood, laminated wood and plywood. The method for treating timber according to the invention mainly consists in contacting one or more compounds according to the invention, or a composition according to the invention ; this includes for example direct application, spraying, dipping, injection or any other suitable means.

The dose of active compound usually applied in the method of treatment according to the invention is generally and advantageously from 10 to 800 g/ha, preferably from 50 to 300 g/ha for applications in foliar treatment. The dose of active substance applied is generally and advantageously from 2 to 200 g per 100 kg of seed, preferably from 3 to 150 g per 100 kg of seed in the case of seed treatment.
It is clearly understood that the doses indicated herein are given as illustrative examples of the method according to the invention. A person skilled in the art will know how to adapt the application doses, notably according to the nature of the plant or crop to be treated.

The fungicide composition according to the invention can also be used in the treatment of genetically modified organisms with the compounds according to the invention or the agrochemical compositions according to the invention. Genetically modified plants are plants into genome of that a heterologous gene encoding a protein of interest has been stably integrated. The expression "heterologous gene encoding a protein of interest" essentially means genes that give the transformed plant new agronomic properties, or genes for improving the agronomic quality of the modified plant.
The compounds or mixtures according to the invention can also be used for the preparation of composition useful to curatively or preventively treat human or animal fungal diseases such as, for example, mycoses, dermatoses, trichophyton diseases and candidiases or diseases caused by *Aspergillus spp*., for example *Aspergillus fumigatus.*

The various aspects of the invention will now be illustrated with reference to the following table of compound examples and the following preparation or efficacy examples.

Table 1 illustrates in a non-limiting manner examples of compounds of formula (I) according to the invention.
In table 1, M+H (Apcl+) means the molecular ion peak plus 1 a.m.u. (atomic mass unit) as observed in mass spectroscopy via positive atmospheric pressure chemical ionisation.

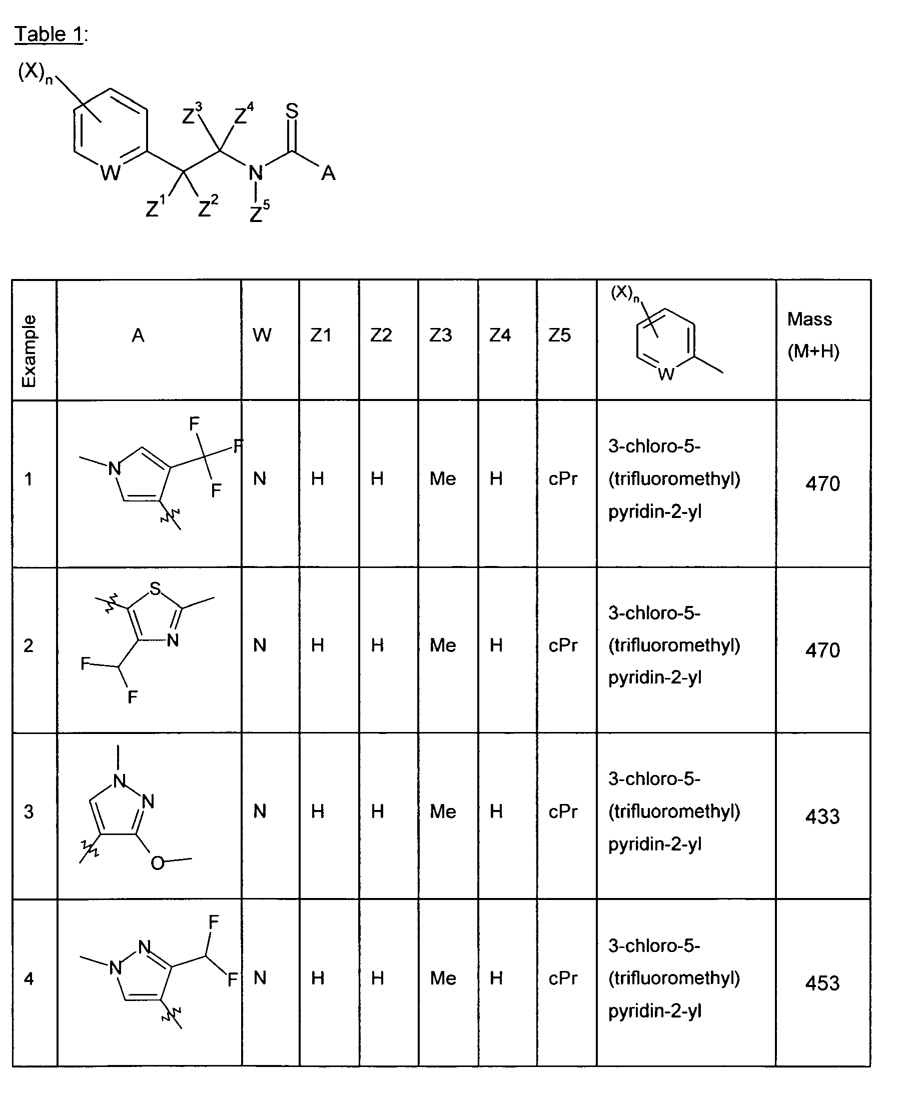

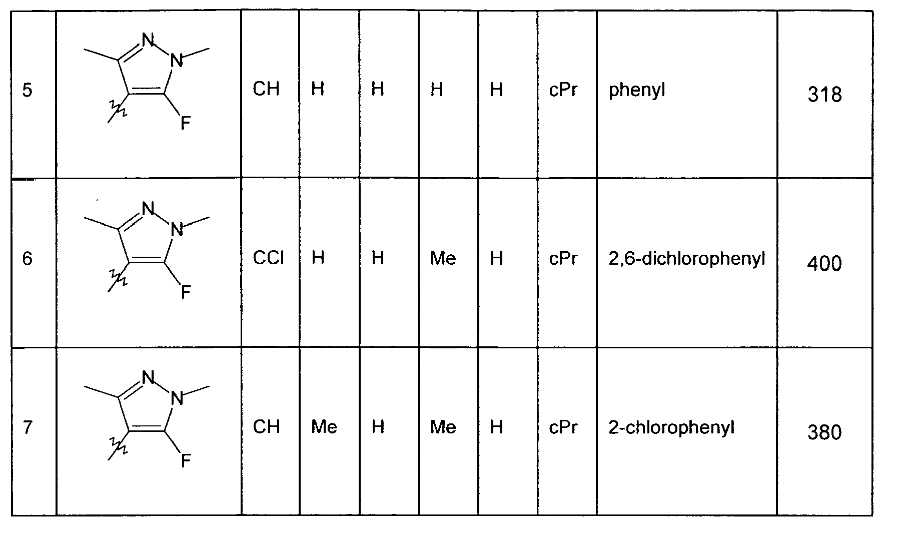

### General preparation example: thionation of amide of formula (II) on Chemspeed apparatus

In a 13 ml Chemspeed vial is weighted 0.27mmole of phosphorous pentasulfide (P₂S₅). 3 ml of a 0.18 molar solution of the amide (II) (0.54mmole) in dioxane is added and the mixture is heated at reflux for two hours. The temperature is then cooled to 80°C and 2.5 ml of water are added. The mixture is heated at 80°C for one more hour. 2 ml of water are then added and the reaction mixture is extracted twice by 4 ml of dichloromethane. The organic phase is deposited on a basic alumina cardridge (2g) and eluted twice by 8 ml of dichloromethane. The solvents are removed and the crude thioamide derivative is analyzed by LCMS and NMR. Insufficiently pure compounds are further purified by preparative LCMS.
In such conditions, N-cyclopropyl-N-[1-(2,6-dichlorophenyl)propan-2-yl]-5-fluoro-1,3-dimethyl -1H-pyrazole-4-carbothioamide (compound 6) is obtained as a yellow oil with a yield of 57% (M+H = 400).

### Example A : in vivo test on Sphaerotheca fuliginea (cucurbits powdery mildew).

The active ingredients tested are prepared by homogenization in a mixture of acetone/tween/DMSO, then diluted with water to obtain the desired active material.
Gherkin plants (Vert petit de Paris variety) in starter cups, sown on a 50/50 peat soil-pozzolana substrate and grown at 20°C/23°C, are treated at the cotyledon Z10 stage by spraying with the aqueous suspension described above. Plants, used as controls, are treated with an aqueous solution not containing the active material.
After 24 hours, the plants are contaminated by spraying them with an aqueous suspension of *Sphaerotheca fuliginea* spores (100 000 spores per ml). The spores are collected from a contaminated plants The contaminated gherkin plants are incubated at about 20°C/25°C and at 60/70% relative humidity.
Grading (% of efficacy) is carried out 12 days after the contamination, in comparison with the control plants.
Under these conditions, good (at least 70%) or total protection is observed at a dose of 500ppm with the following compounds: 1, 3, 4, 5, 6 and 7

### Example B : in vivo test on Botrytis cinerea (Grey mould)

The active ingredients tested are prepared by homogenization in a mixture of acetone/Tween/DMSO, then diluted with water to obtain the desired active material Gherkin plants (Vert petit de Paris variety), sown on a 50/50 peat soil-pozzolana substrate in starter cups and grown at 18- 20°C, are treated at the cotyledon Z11 stage by spraying with the active ingredient prepared as described above.
Plants, used as controls, are treated with an aqueous solution not containing the active material.
After 24 hours, the plants are contaminated by depositing drops of an aqueous suspension of *Botrytis cinerea* spores (150,000 spores per ml) on upper surface of the leaves. The spores are collected from a 15-day-old culture and are suspended in a nutrient solution composed of:
- 20 g/L of gelatine;
- 50 g/L of D-fructose;
- 2 g/L of NH₄NO₃;
- 1 g/L of KH₂PO₄.

The contaminated cucumber plants are settled for 5/7 days in a climatic room at 15-11°C (day/night) and at 80% relative humidity.
Grading is carried out 5/7 days after the contamination, in comparison with the control plants. Under these conditions, good (at least 70%) or total protection is observed at a dose of 500 ppm with the following compounds: 6

### Example C : in vivo test on Pyrenoohora teres (Barley Net blotch)

The active ingredients tested are prepared by homogenization in a mixture of acetone/Tween/DMSO, then diluted with water to obtain the desired active material concentration.
Barley plants (Express variety), sown on a 50/50 peat soil-pozzolana substrate in starter cups and grown at 12°C, are treated at the 1-leaf stage (10 cm tall) by spraying with the active ingredient prepared as described above.
Plants, used as controls, are treated with an aqueous solution not containing the active material.

After 24 hours, the plants are contaminated by spraying them with an aqueous suspension of *Pyrenophora teres* spores (12,000 spores per ml). The spores are collected from a 12-day-old culture. The contaminated barley plants are incubated for 24 hours at about 20°C and at 100% relative humidity, and then for 12 days at 80% relative humidity.
Grading is carried out 12 days after the contamination, in comparison with the control plants. Under these conditions, good (at least 70%) is observed at a dose of 500 ppm with the following compounds: 1, 3, 4, 5, 6 and 7

### Example D : in vivo test on Puccinia recondita (Brown rust)

The active ingredients tested are prepared by homogenization in a mixture of acetone/tween/DMSO, then diluted with water to obtain the desired active material.
Wheat plants (Scipion variety) sown on 50/50 peat soil-pozzolana substrate in starter cups and grown at 12°C, are treated at the 1-leaf stage (10 cm tall) by spraying with the aqueous suspension described above.
Plants, used as controls, are treated with an aqueous solution not containing the active material.
After 24 hours, the plants are contaminated by spraying the leaves with an aqueous suspension of *Puccinia recondita* spores (100,000 spores per ml). The spores are collected from a 10-day-old contaminated wheat and are suspended in water containing 2.5 ml/I of tween 80 10%. The contaminated wheat plants are incubated for 24 hours at 20°C and at 100% relative humidity, and then for 10 days at 20°C and at 70% relative humidity.
Grading is carried out 10 days after the contamination, in comparison with the control plants. Under these conditions, good (at least 70%) or total protection is observed at a dose of 500ppm with the following compounds: 6

### Example E : in vivo test on Mycosphaerella graminicola (Wheat Leaf Spot)

The active ingredients tested are prepared by homogenization in a mixture of acetone/tween/DMSO, then diluted with water to obtain the desired active material concentration.
Wheat plants (Scipion variety), sown on a 50/50 peat soil-pozzolana substrate in starter cups and grown at 12°C, are treated at the 1-leaf stage (10 cm tall) by spraying with the aqueous suspension described above. Plants, used as controls, are treated with an aqueous solution not containing the active material.
After 24 hours, the plants are contaminated by spraying them with an aqueous suspension of *Mycosphaerella graminicola* spores (500 000 spores per ml). The spores are collected from a 7-day-old culture. The contaminated wheat plants are incubated for 72 hours at 18°C and at 100% relative humidity, and then for 21 to 28 days at 90% relative humidity.
Grading (% of efficacy) is carried out 21 to 28 days after the contamination, in comparison with the control plants.
Under these conditions, good (at least 70%) or total protection is observed at a dose of 500ppm with the following compounds: 1, 3, 4 and 5

## Claims

1. A compound of formula (I) 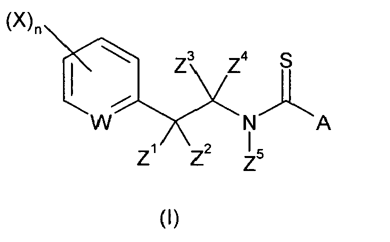 wherein
• A represents a carbo-linked, unsaturated or partially saturated, 5-membered heterocyclyl group that can be substituted by up to four groups R ;
• W represents a nitrogen atom or a carbon atom substituted by a hydrogen atom or by the group X ;
• n represents 0, 1, 2, 3 or 4 ;
• X represents a halogen atom, a nitro group, a cyano group, a hydroxy group, an amino group, a sulfanyl group, a pentafluoro-λ⁶-sulfanyl group, a formyl group, a formyloxy group, a formylamino group, a carboxy group, a carbamoyl group, a N-hydroxycarbamoyl group, a carbamate group, a (hydroxyimino)-C₁-C₆-alkyl group, a C₁-C₈-halogenoalkyl having 1 to 5 halogen atoms, a C₁-C₈-alkyl, a C₁-C₈-arylalkyl, a (C₃-C₇-cycloalkyl)-C₁-C₈-alkyl, a C₂-C₈-alkenyl, a C₂-C₈-arylalkenyl, a (C₃-C₇-cycloalkyl)-C₂-C₈-alkenyl, a C₂-C₈-alkynyl, a C₂-C₈-arylalkynyl, a (C₃-C₇-cycloalkyl)-C₂-C₈-alkynyl, a C₁-C₈-alkylamino, a di-C₁-C₈-alkylamino, a C₁-C₈-alkoxy, a C₁-C₈-halogenoalkoxy having 1 to 5 halogen atoms, a C₁-C₈-alkylsulfanyl, a C₁-C₈-halogenoalkylsulfanyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₃-C₈-cycloalkyl, a C₃-C₈-halogenocycloalkyl having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a N-C₁-C₈-alkyloxycarbamoyl, a C₁-C₈-alkoxycarbamoyl, a N-C₁-C₈-alkyl-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl, a C₁-C₈-halogenoalkylsulphonyl having 1 to 5 halogen atoms, a C₁-C₆-alkoxyimino, a (C₁-C₆-alkoxyimino)-C₁-C₆-alkyl, a (C₁-C₆-alkenyloxyimino)-C₁-C₆-alkyl, a (C₁-C₆-alkynyloxyimino)-C₁-C₆-alkyl, a (benzyloxyimino)-C₁-C₆-alkyl, a tri(C₁-C₈)alkylsilyl, a tri(C₁-C₈)alkylsilyl-C₁-C₈-alkyl, a benzyloxy that can be substituted by up to 5 groups Q ; a benzylsulfanyl that can be substituted by up to 5 groups Q ; a benzylamino that can be substituted by up to 5 groups Q ; a phenyl that can be substituted by up to 5 groups Q ; a naphtyl that can be substituted by up to 6 groups Q ; a phenoxy that can be substituted by up to 5 groups Q ; a phenylamino that can be substituted by up to 5 groups Q ; a phenylsulfanyl that can be substituted by up to 5 groups Q ; a phenylmethylene that can be substituted by up to 7 groups Q ; a pyridinyl that can be substituted by up to four groups Q or a pyridinyloxy that can be substituted by up to four groups Q ;
• Z¹ and Z² independently represent a hydrogen atom, a halogen atom, a cyano group, a hydroxy group, an amino group, a sulfanyl group, a formyl group, a formyloxy group, a formylamino group, a carboxy group, a carbamoyl group, a N-hydroxycarbamoyl group, a carbamate group, a (hydroxyimino)-C₁-C₈-alkyl group, a C₁-C₈-alkyl, a C₂-C₈-alkenyl, a C₂-C₈-alkynyl, a C₁-C₈-alkylamino, a di-C₁-C₈-alkylamino, a C₁-C₈-alkoxy, a C₁-C₈-halogenoalkyl having 1 to 5 halogen atoms, a C₁-C₈-halogenoalkoxy having 1 to 5 halogen atoms, a C₁-C₈-alkylsulfanyl, a C₁-C₈-halogenoalkylsulfanyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₃-C₇-cycloalkyl, a C₃-C₇-halogenocycloalkyl having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a (N-C₁-C₈-alkyl)oxycarbamoyl, a C₁-C₈-alkoxycarbamoyl, a (N-C₁-C₈-alkyl)-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl or a C₁-C₈-halogenoalkylsulphonyl having 1 to 5 halogen atoms,
• Z³ and Z⁴ independently represent a hydrogen atom, a halogen atom, a cyano group, a hydroxy group, an amino group, a sulfanyl group, a formyl group, a formyloxy group, a formylamino group, a carboxy group, a carbamoyl group, a N-hydroxycarbamoyl group, a carbamate group, a (hydroxyimino)-C₁-C₈-alkyl group, a C₁-C₈-alkyl, a C₂-C₈-alkenyl, a C₂-C₈-alkynyl, a C₁-C₈-alkylamino, a di-C₁-C₈-alkylamino, a C₁-C₈-alkoxy, a C₁-C₈-halogenoalkyl having 1 to 5 halogen atoms, a C₁-C₈-halogenoalkoxy having 1 to 5 halogen atoms, a C₁-C₈-alkylsulfanyl, a C₁-C₈-halogenoalkylsulfanyl having 1 to 5 halogen atoms, a C₂-C₈-alkenyloxy, a C₂-C₈-halogenoalkenyloxy having 1 to 5 halogen atoms, a C₃-C₈-alkynyloxy, a C₃-C₈-halogenoalkynyloxy having 1 to 5 halogen atoms, a C₃-C₇-cycloalkyl, a C₃-C₇-halogenocycloalkyl having 1 to 5 halogen atoms, a C₁-C₈-alkylcarbonyl, a C₁-C₈-halogenoalkylcarbonyl having 1 to 5 halogen atoms, a C₁-C₈-alkylcarbamoyl, a di-C₁-C₈-alkylcarbamoyl, a (N-C₁-C₈-alkyl)oxycarbamoyl, a C₁-C₈-alkoxycarbamoyl, a (N-C₁-C₈-alkyl)-C₁-C₈-alkoxycarbamoyl, a C₁-C₈-alkoxycarbonyl, a C₁-C₈-halogenoalkoxycarbonyl having 1 to 5 halogen atoms, a C₁-C₈-alkylcarbonyloxy, a C₁-C₈-halogenoalkylcarbonyloxy having 1 to 5 halogen atoms, a C₁-C₈-alkylcarbonylamino, a C₁-C₈-halogenoalkylcarbonylamino having 1 to 5 halogen atoms, a C₁-C₈-alkylaminocarbonyloxy, a di-C₁-C₈-alkylaminocarbonyloxy, a C₁-C₈-alkyloxycarbonyloxy, a C₁-C₈-alkylsulphenyl, a C₁-C₈-halogenoalkylsulphenyl having 1 to 5 halogen atoms, a C₁-C₈-alkylsulphinyl, a C₁-C₈-halogenoalkylsulphinyl having 1 to 5 halogen atoms, a C₁-C₈-alkylsulphonyl or a C₁-C₈-halogenoalkylsulphonyl having 1 to 5 halogen atoms, With the provisio that when W is a nitrogen atom, then a least one of the four substituents Z₁, Z₂, Z₃ or Z₄ is not a hydrogen atom;
• Z⁵ represents a non-substituted C₃-C₇-cycloalkyl or a C₃-C₇-cycloalkyl substituted by up to 10 atoms or groups that can be the same or different and that can be selected in the list consisting of halogen atoms ; cyano ; C₁-C₈-alkyl ; C₁-C₈-halogenoalkyl comprising up to 9 halogen atoms that can be the same or different ; C₁-C₈-alkoxy ; C₁-C₈-halogenoalkoxy comprising up to 9 halogen atoms that can be the same or different ; C₁-C₈-alkoxycarbonyl ; C₁-C₈-halogenoalkoxycarbonyl comprising up to 9 halogen atoms that can be the same or different ; C₁-C₈-alkylaminocarbonyl ; or di-C₁-C₈-alkylaminocarbonyl ;
• Q independently represents a halogen atom ; cyano ; nitro ; C₁-C₈-alkyl ; C₁-C₈-alkoxy ; C₁-C₈-alkylsulfanyl ; C₁-C₈-halogenoalkyl comprising up to 9 halogen atoms that can be the same or different ; C₁-C₈-halogenoalkoxy comprising up to 9 halogen atoms that can be the same or different ; tri(C₁-C₈)alkylsilyl and tri(C₁-C₈)alkylsilyl-C₁-C₈-alkyl ; C₁-C₈-alkoxyimino ; or (C₁-C₈-alkoxyimino)- C₁-C₈-alkyl ;
• R independently represents a hydrogen atom ; halogen atom ; cyano ; nitro ; amino ; sulfanyl ; pentafluoro-λ-6-sulfanyl ; C₁-C₈-alkylamino ; di-C₁-C₈-alkylamino ; tri(C₁-C₈-alkyl)silyl ; C₁-C₈-alkylsulfanyl ; C₁-C₈-halogenoalkylsulfanyl comprising up to 9 halogen atoms that can be the same or different ; C₁-C₈-alkyl ; C₁-C₈-halogenoalkyl comprising up to 9 halogen atoms that can be the same or different ; C₂-C₈-alkenyl ; C₂-C₈-halogenoalkenyl comprising up to 9 halogen atoms that can be the same or different ; C₂-C₈-alkynyl ; C₂-C₈-halogenoalkynyl comprising up to 9 halogen atoms that can be the same or different ; C₁-C₈-alkoxy ; C₁-C₈-halogenoalkoxy comprising up to 9 halogen atoms that can be the same or different ; C₂ C₈-alkenyloxy ; C₂-C₈-alkynyloxy ; C₃-C₇-cycloalkyl ; C₃-C₇-cycloalkyl- C₁-C₈-alkyl ; C₁-C₈-alkylsulphinyl ; C₁-C₈-alkylsulphonyl ; C₁-C₈alkoxyimino ; (C₁-C₈-alkoxyimino)-C₁-C₈-alkyl (benzyloxyimino)-C₁-C₈-alkyl ; phenoxy ; benzyloxy ; benzylsulfanyl ; benzylamino ; naphtyl ; halogenophenoxy comprising up to 9 halogen atoms that can be the same or different ; C₁-C₈-alkylcarbonyl ; C₁-C₈-halogenoalkylcarbonyl comprising up to 9 halogen atoms that can be the same or different ; C₁-C₈-alkoxycarbonyl ; C₁-C₈-halogenoalkoxycarbonyl comprising up to 9 halogen atoms that can be the same or different; C₁-C₈-alkylaminocarbonyl ; or di-C₁-C₈-alkylaminocarbonyl ;
as well as its salts, N-oxydes, metallic complexes, metalloidic complexes and optically active isomers.

2. A compound according to claim 1 wherein A is selected in the list consisting of:
- a heterocycle of formula (A¹) 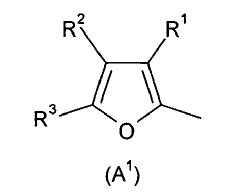 wherein:
R¹ to R³ that can be the same or different represent a hydrogen atom ; a halogen atom ; C₁-C₅-alkyl ; C₁-C₅-halogenoalkyl comprising up to 9 halogen atoms that can be the same or different ; C₁-C₅-alkoxy or C₁-C₅-halogenoalkoxy comprising up to 9 halogen atoms that can be the same or different;
- a heterocycle of formula (A²) 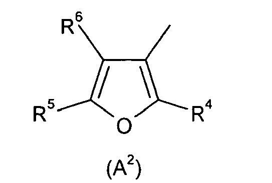 wherein :
R⁴ to R⁶ that can be the same or different represent a hydrogen atom ; a halogen atom ; C₁-C₅-alkyl ; C₁-C₅-halogenoalkyl comprising up to 9 halogen atoms that can be the same or different ; C₁-C₅-alkoxy or C₁-C₅-halogenoalkoxy comprising up to 9 halogen atoms that can be the same or different;
- a heterocycle of formula (A³) 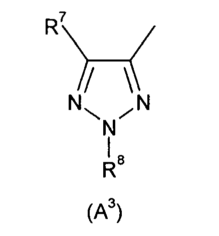 wherein :
R⁷ represents a hydrogen atom ; a halogen atom ; C₁-C₅-alkyl ; C₁-C₅-halogenoalkyl comprising up to 9 halogen atoms that can be the same or different ; C₁-C₅-alkoxy or C₁-C₅-halogenoalkoxy comprising up to 9 halogen atoms that can be the same or different;
R⁸ represents a hydrogen atom or a C₁-C₅-alkyl ;
- a heterocycle of formula (A⁴) 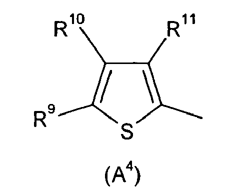 wherein :
R⁹ to R¹¹ that can be the same or different represent a hydrogen atom ; a halogen atom ; C₁-C₅-alkyl ; amino ; C₁-C₅-alkoxy ; C₁-C₅-alkylsulphanyl ; C₁-C₅-halogenoalkyl comprising up to 9 halogen atoms that can be the same or different or C₁-C₅-halogenoalkoxy comprising up to 9 halogen atoms that can be the same or different;
- a heterocycle of formula (A⁵) 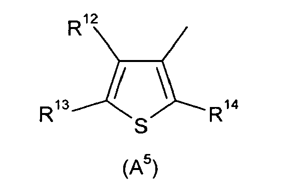 wherein :
R¹² and R¹³ that can be the same or different represent a hydrogen atom ; a halogen atom ; C₁-C₅-alkyl ; C₁-C₅-alkoxy ; amino ; C₁-C₅-halogenoalkyl comprising up to 9 halogen atoms that can be the same or different or C₁-C₅-halogenoalkoxy comprising up to 9 halogen atoms that can be the same or different ;
R¹⁴ represents a hydrogen atom ; a halogen atom ; C₁-C₅-alkyl ; C₁-C₅-alkoxy ; amino ; C₁-C₅-halogenoalkyl comprising up to 9 halogen atoms that can be the same or different or C₁-C₅-halogenoalkoxy comprising up to 9 halogen atoms that can be the same or different;
- a heterocycle of formula (A⁶) 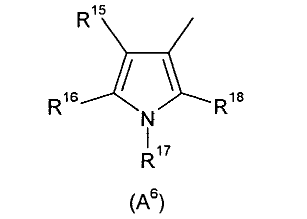 wherein :
R¹⁵ represents a hydrogen atom ; a halogen atom ; a cyano ; C₁-C⁵-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different;
R¹⁶ and R¹⁸ that can be the same or different represent a hydrogen atom ; a halogen atom ; C₁-C₅-alkoxycarbonyl ; C₁-C₅-alkyl ; C₁-C₅-halogenoalkoxy comprising up to 9 halogen atoms that can be the same or different or C₁-C₅-halogenoalkyl comprising up to 9 halogen atoms that can be the same or different;
R¹⁷ represent a hydrogen atom or C₁-C₅-alkyl ;
- a heterocycle of formula (A⁷) 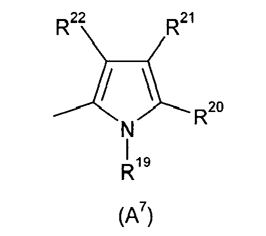 wherein :
R¹⁹ represents a hydrogen atom or a C₁-C₅-alkyl
R²⁰ to R²² that can be the same or different represent a hydrogen atom ; a halogen atom ; C₁-C₅-alkyl or C₁-C₅-halogenoalkyl comprising up to 9 halogen atoms that can be the same or different ;
- a heterocycle of formula (A⁸) 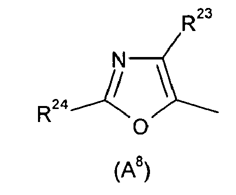 wherein :
R²³ represents a hydrogen atom ; a halogen atom ; C₁-C₅-alkyl or C₁-C₅-halogenoalkyl comprising up to 9 halogen atoms that can be the same or different;
R²⁴ represents a hydrogen atom or C₁-C₅-alkyl or C₁-C₅-halogenoalkyl comprising up to 9 halogen atoms that can be the same or different ;
- a heterocycle of formula (A⁹) 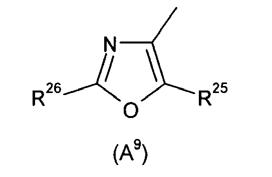 wherein :
R²⁵ represents a hydrogen atom ; a halogen atom ; C₁-C₅-alkyl or C₁-C₅-halogenoalkyl comprising up to 9 halogen atoms that can be the same or different;
R²⁶ represents a hydrogen atom ; C₁-C₅-alkyl or C₁-C₅-halogenoalkyl comprising up to 9 halogen atoms that can be the same or different;
- a heterocycle of formula (A¹⁰) 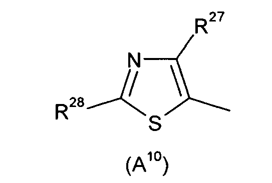 wherein :
R²⁷ represents a hydrogen atom ; a halogen atom ; C₁-C₅-alkyl or C₁-C₅-halogenoalkyl comprising up to 9 halogen atoms that can be the same or different ;
R²⁸ represents a hydrogen atom ; a halogen atom ; C₁-C₅-alkyl ; C₁-C₅-halogenoalkyl comprising up to 9 halogen atoms that can be the same or different ; C₁-C₅-halogenoalkoxy comprising up to 9 halogen atoms that can be the same or different ; amino ; C₁-C₅-alkylamino or di(C₁-C₈-alkyl)amino ;
- a heterocycle of formula (A¹¹) 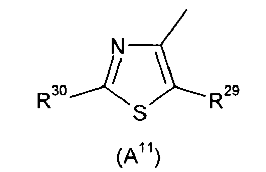 wherein :
R²⁹ represents a hydrogen atom ; a halogen atom ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different ;
R³⁰ represents a hydrogen atom ; a halogen atom ; C₁-C₅-alkyl ; C₁-C₅-halogenoalkyl comprising up to 9 halogen atoms that can be the same or different ; C₁-C₅-halogenoalkoxy comprising up to 9 halogen atoms that can be the same or different ; amino ; C₁-C₅-alkylamino or di(C₁-C₅-alkyl)amino ;
- a heterocycle of formula (A¹²) 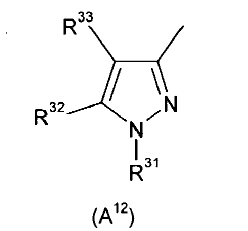 wherein :
R³¹ represents a hydrogen atom or a C₁-C₅-alkyl
R³² represents a hydrogen atom ; a halogen atom ; C₁-C₅-alkyl or C₁-C₅-halogenoalkyl comprising up to 9 halogen atoms that can be the same or different;
R³³ represents a hydrogen atom ; a halogen atom ; a nitro ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different;
- a heterocycle of formula (A¹³) 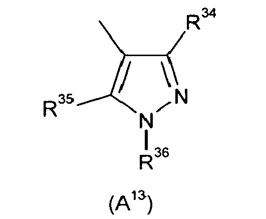 wherein :
R³⁴ represents a hydrogen atom ; a halogen atom ; C₁-C₅-alkyl ; C₃-C₅-cycloalkyl C₁-C₅-halogenoalkyl comprising up to 9 halogen atoms that can be the same or different ; C₁-C₅-alkoxy ; C₂-C₅-alkynyloxy or C₁-C₅-halogenoalkoxy comprising up to 9 halogen atoms that can be the same or different;
R³⁵ represents a hydrogen atom ; a halogen atom ; C₁-C₅-alkyl ; a cyano ; C₁-C₅-alkoxy ; C₁-C₅-alkylsulphanyl ; C₁-C₅-halogenoalkyl comprising up to 9 halogen atoms that can be the same or different ; C₁-C₅-halogenoalkoxy comprising up to 9 halogen atoms that can be the same or different; amino ; C₁-C₅-alkylamino or di(C₁-C₅-alkyl)amino ;
R³⁶ represents a hydrogen atom or C₁-C₅-alkyl ;
- a heterocycle of formula (A¹⁴) 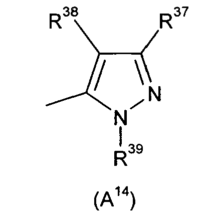 wherein :
R³⁷ and R³⁸ that can be the same or different represent a hydrogen atom ; a halogen atom ; C₁-C₅-alkyl ; C₁-C₅-halogenoalkyl comprising up to 9 halogen atoms that can be the same or different; C₁-C₅-alkoxy or a C₁-C₅- alkylsulphanyl ;
R³⁹ represents a hydrogen atom or C₁-C₅-alkyl ;
- a heterocycle of formula (A¹⁵) 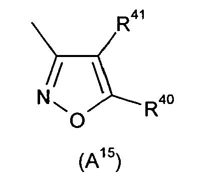 wherein :
R⁴⁰ and R⁴¹ that can be the same or different represent a hydrogen atom ; a halogen atom ; C₁-C₅-alkyl or C₁-C₅-halogenoalkyl comprising up to 9 halogen atoms that can be the same or different ;
- a heterocycle of formula (A¹⁶) 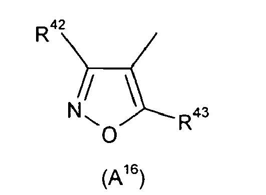 wherein :
R⁴² and R⁴³ that can be the same or different represent a hydrogen atom ; a halogen atom ; C₁-C₅-alkyl ; C₁-C₅-halogenoalkyl comprising up to 9 halogen atoms that can be the same or different or amino;
- a heterocycle of formula (A¹⁷) 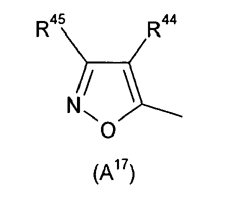 wherein :
R⁴⁴ and R⁴⁵ that can be the same or different represent a hydrogen atom ; a halogen atom ; C₁-C₅-alkyl or C₁-C₅-halogenoalkyl comprising up to 9 halogen atoms that can be the same or different ;
- a heterocycle of formula (A¹⁸) 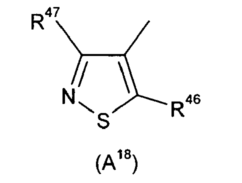 wherein :
R⁴⁷ represents a hydrogen atom ; a halogen atom ; C₁-C₅-alkyl or C₁-C₅-halogenoalkyl comprising up to 9 halogen atoms that can be the same or different;
R⁴⁶ represents a hydrogen atom ; a halogen atom ; C₁-C₅-alkyl ; C₁-C₅-halogenoalkyl comprising up to 9 halogen atoms that can be the same or different or C₁-C₅-alkylsulfanyl ;
- a heterocycle of formula (A¹⁹) 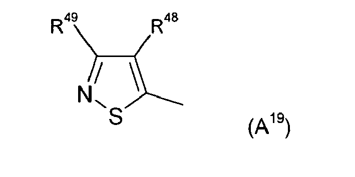 wherein :
R⁴⁹ and R⁴⁸ that can be the same or different represent a hydrogen atom ; a halogen atom ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different ;
- a heterocycle of formula (A²⁰) 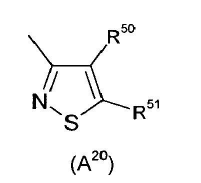 wherein :
R⁵⁰ and R⁵¹ that can be the same or different represent a hydrogen atom ; a halogen atom ; C₁-C₅-alkyl ; C₁-C₅-alkoxy ; C₁-C₅-halogenoalkoxy comprising up to 9 halogen atoms that can be the same or different or C₁-C₅-halogenoalkyl comprising up to 9 halogen atoms that can be the same or different;
- a heterocycle of formula (A²¹) 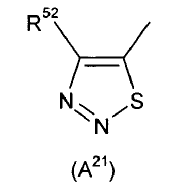 wherein :
R⁵² represents a hydrogen atom ; a halogen atom ; C₁-C₅-alkyl or C₁-C₅-halogenoalkyl comprising up to 9 halogen atoms that can be the same or different.
- a heterocycle of formula (A²²) 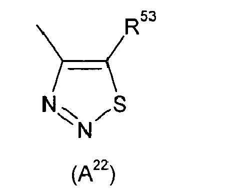 wherein :
R⁵³ represents a hydrogen atom ; a halogen atom ; C₁-C₅-alkyl or C₁-C₅-halogenoalkyl comprising up to 9 halogen atoms that can be the same or different.
- a heterocycle of formula (A²³) 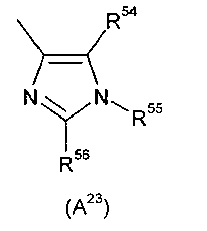 wherein :
R⁵⁴ and R⁵⁶ that can be the same or different represent a hydrogen atom ; a halogen atom ; C₁-C₅-alkyl or C₁-C₅-halogenoalkyl comprising up to 9 halogen atoms that can be the same or different ;
R⁵⁵ represents a hydrogen atom or C₁-C₅-alkyl ;
- a heterocycle of formula (A²⁴) 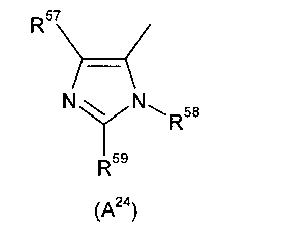 wherein :
R⁵⁷ and R⁵⁹ that can be the same or different represent a hydrogen atom ; a halogen atom ; C₁-C₅-alkyl or C₁-C₅-halogenoalkyl comprising up to 9 halogen atoms that can be the same or different ;
R⁵⁸ represents a hydrogen atom or C₁-C₅-alkyl ;
- a heterocycle of formula (A²⁵) 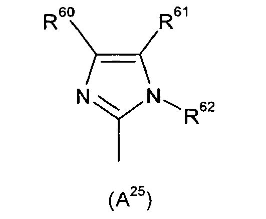 wherein :
R⁶⁰ and R⁶¹ that can be the same or different represent a hydrogen atom ; a halogen atom ; C₁-C₅-alkyl or C₁-C₅-halogenoalkyl comprising up to 9 halogen atoms that can be the same or different ;
R⁶² represents a hydrogen atom or C₁-C₅-alkyl ;
- a heterocycle of formula (A²⁶) 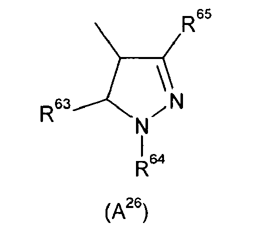 wherein :
R⁶⁵ represents a hydrogen atom ; a halogen atom ; C₁-C₅-alkyl ; C₃-C₅-cycloalkyl ; C₁-C₅-halogenoalkyl comprising up to 9 halogen atoms that can be the same or different ; C₁-C₅-alkoxy ; C₂-C₅-alkynyloxy or C₁-C₅-halogenoalkoxy comprising up to 9 halogen atoms that can be the same or different;
R⁶³ represents a hydrogen atom ; a halogen atom ; C₁-C₅-alkyl ; a cyano ; C₁-C₅-alkoxy ; C₁-C₅-alkylsulphanyl ; C₁-C₅-halogenoalkyl comprising up to 9 halogen atoms that can be the same or different ; C₁-C₅-halogenoalkoxy comprising up to 9 halogen atoms that can be the same or different; amino ; C₁-C₅-alkylamino or di(C₁-C₅-alkyl)amino ;
R⁶⁴ represents a hydrogen atom or C₁-C₅-alkyl.

3. A compound according to claim 2 wherein A is selected in the list consisting of A²; A⁶; A¹⁰ and A¹³.

4. A compound according to claim 3 wherein A represents A¹³, wherein R³⁴ represents a C₁-C₅-alkyl, C₁-C₅-halogenoalkyl comprising up to 9 halogen atoms that can be the same or different ; or C₁-C₅-alkoxy ; wherein R³⁵ represents a hydrogen atom or a halogen atom and wherein R³⁶ represents a C₁-C₅-alkyl.

5. A compound according to claim 1 wherein W represents a nitrogen atom or a carbon atom substituted by a hydrogen atom or a halogen atom.

6. A compound according to claim 1 wherein n represents 0, 1 or 2.

7. A compound according to claim 1 wherein X independently represents a halogen atom ; C₁-C₈-alkyl ; C₁-C₈-halogenoalkyl comprising up to 9 halogen atoms that can be the same or different ; tri(C₁-C₈-alkyl)silyl ; C₁-C₈-alkoxy or C₁-C₈-halogenoalkoxy comprising up to 9 halogen atoms that can be the same or different; or wherein two consecutive substituents X together with the phenyl ring form a substituted or non substituted 1,3-benzodioxolyl ; 1,2,3,4-tetrahydro-quinoxalinyl ; 3,4-dihydro-2H-1,4-benzoxazinyl ; 1,4-benzodioxanyl ; indanyl ; 2,3-dihydrobenzofuranyl ; or indolinyl.

8. A compound according to claim 1 wherein Z¹ and Z² independently represent a hydrogen atom, a halogen atom, a C₁-C₈-alkyl, a C₁-C₈-alkoxy or a C₁-C₈-halogenoalkyl having 1 to 5 halogen atoms.

9. A compound according to claim 1 wherein Z³ and Z⁴ independently represent a hydrogen atom, a C₁-C₈-alkyl, a C₁-C₈-alkoxy or a C₁-C₈-halogenoalkyl having 1 to 5 halogen atoms.

10. A compound according to claim 1 wherein Z⁵ represents a C₃-C₇ cycloalkyl substituted by up to 10 groups or atoms that can be the same or different and that can be selected in the list consisting of halogen atoms; C₁-C₈-alkyl ; C₁-C₈-halogenoalkyl comprising up to 9 halogen atoms that can be the same or different ; C₁-C₈-alkoxy or C₁-C₈-halogenoalkoxy comprising up to 9 halogen atoms that can be the same or different;

11. A compound according to claim 10 wherein Z⁵ represents a non-substituted C₃-C₇-cycloalkyl, preferably a cyclopropyl.

12. A compound according to claim 1 wherein R independently represents a hydrogen atom ; halogen atom ; cyano ; C₁-C₈-alkylamino ; di-C₁-C₈-alkylamino ; tri(C₁-C₈-alkyl)silyl ; C₁-C₈-alkyl ; C₁-C₈-halogenoalkyl comprising up to 9 halogen atoms that can be the same or different ; C₁-C₈-alkoxy ; C₁-C₈-halogenoalkoxy comprising up to 9 halogen atoms that can be the same or different; C₁-C₈-alkylsulfanyl ; amino, hydroxyl ; nitro ; C₁-C₈-alkoxycarbonyl ; or C₂-C₈-alkynyloxy.

13. A fungicide composition comprising, as an active ingredient, an effective amount of a compound of formula (I) according to claims 1 to 12 and an agriculturally acceptable support, carrier or filler.

14. A method for controlling phytopathogenic fungi of crops, **characterized in that** an agronomically effective and substantially non-phytotoxic quantity of a compound according to claims 1 to 12 or a composition according to claim 13 is applied to the soil where plants grow or are capable of growing, to the leaves and/or the fruit of plants or to the seeds of plants.
